# EUROPEAN PATENT APPLICATION

(11) **EP 4 821 470 A1**
(43) Date of publication of application: **14.10.2026**
(21) Application number: 26171295.4
(22) Date of filing: 08.04.2026
(51) Int. Cl.: A61F 13/04, A61F 5/01, A61F 5/058, A61F 13/10

(54) **A MEDICAL CAST**

(30) Priority: 08.04.2025 GB 202505281
(71) Applicant: Additive Design Ltd, Leeds LS1 2LH (GB)
(72) Inventor: ABDI, Alireza, Leeds, LS1 2LH (GB); WALSHAW, Chris, Leeds, LS1 2LH (GB); CHAUHAN, Gaurav, Leeds, LS1 2LH (GB)
(74) Representative: HGF

(57) **Abstract**

A cast (100) for supporting the lower forearm (LFA), wrist (WR) and at least a metacarpal portion (MP) of a hand of a user, comprising: a first cast portion (110) that extends along a first face (PF, DF) of the lower forearm (LFA), wrist (WR) and metacarpal portion (MP) of the hand of the user, wherein the first face is the planar face (PF) or the dorsal face (DF); and a second cast portion (120) that extends along the opposed second face (DF, PF) of the lower forearm (LFA), wrist (WR) and metacarpal portion (MP) of the hand of the user, wherein the first cast portion (110) extends across both first and second faces (PF, DF) of one or more of the finger metacarpophalangeal joints (FMJ), the finger proximal phalanx bones (FPP), the finger intermediate phalanx bones (FIP) and the finger distal phalanx bones (FDP) of the hand of the user, and wherein in a closed configuration, the first cast portion (110) and second cast portion (112) meet along at least part of a radial join (130R) and an ulnar join (130U) that respectively extend along the radial side (RS) and the ulnar side (US) of at least the lower forearm (LFA) and wrist (WR) of a user, the first cast portion (110) and second cast portion (112) meet at a lateral join (130L) that extends between the radial join (130R) and the ulnar join (130U), wherein one or more releasable hinge connectors (140H) are provided at the lateral join (130L) to releasably connect together the first cast portion (110) and the second cast portion (112), each of the one or more releasable hinge connectors (140H) comprising a first hinge part (140H1) and a cooperating second hinge part (140H2) being provided on different ones of the first cast portion (110) and the second cast portion (112).

## Description

### TECHNICAL FIELD

The present application relates to medical casts.

### BACKGROUND

Fixed medical casts that are moulded around limbs are commonly used to immobilise bones and soft tissues during healing. They are commonly formed from fibreglass or plaster. Such casts are typically bulky and being fixed, they hinder moisture management and hygiene.

Less bulky casts (also known as splints) may be constructed by moulding thermosetting plastic sheeting. However, thermoset plastic moulding is unsuitable for conveniently manufacturing casts that extend fully around the lower forearm, wrist and at least part of the hand. Further, such casts are held in place by wrapping with bandage, hook and loop fastenings, can be difficult to fit or remove without assistance from a third party, and can be unsuitable for forming a cast that may be conveniently used on a wrist and hand with involuntarily contracted muscles.

There remains a need for a relatively lightweight openable cast that may more easily be fitted without assistance from a third party. There also remains a need for a cast that be used by patients with involuntarily clenched wrist and hand muscles to provide a therapeutic benefit.

### SUMMARY OF THE DISCLOSURE

According to the present disclosure, there is provided a cast in accordance with the appended claims.

According to a first aspect, there is provided a cast 100 for supporting the lower forearm LFA, wrist WR and at least a metacarpal portion MP of a hand of a user, comprising:
a first cast portion 110 that extends along a first face PF, DF of the lower forearm LFA, wrist WR and metacarpal portion MP of the hand of the user, wherein the first face is the planar face PF or the dorsal face DF; and
a second cast portion 120 that extends along the opposed second face DF, PF of the lower forearm LFA, wrist WR and metacarpal portion MP of the hand of the user,

wherein the first cast portion 110 extends across both first and second faces PF, DF of one or more of the finger metacarpophalangeal joints FMJ, the finger proximal phalanx bones FPP, the finger intermediate phalanx bones FIP and the finger distal phalanx bones FDP of the hand of the user, and
wherein in a closed configuration, the first cast portion 110 and second cast portion 112 meet at (e.g. abut along part or all of) a radial join 130R and an ulnar join 130U that respectively extend along the radial side RS and the ulnar side US of at least the lower forearm LFA and wrist WR of a user,
the first cast portion 110 and second cast portion 112 meet at (e.g. abut along part or all of) a lateral join 130L that extends between the radial join 130R and the ulnar join 130U,
wherein one or more releasable hinge connectors 140H are provided at the lateral join 130L to releasably connect together the first cast portion 110 and the second cast portion 112, each of the one or more releasable hinge connectors 140H comprising a first hinge part 140H1 and a cooperating second hinge part 140H2 being provided on different ones of the first cast portion 110 and the second cast portion 112.

According to a second aspect, there is provided a cast 100 for supporting the lower forearm LFA, wrist WR and hand MP, FG, TH of a user,
comprising a releasably connectable first cast portion 110 and second cast portion 112 for enclosing the lower forearm LFA, wrist WR, and hand,
wherein in a closed configuration, the first cast portion 110 and second cast portion 112 abut along part or all of a radial join 130R and an ulnar join 130U that respectively extend along the radial side RS and the ulnar side US of at least the lower forearm LFA and wrist WR of a user,
wherein the distal end 100DE of the cast 100 extends to the fingertips FT and thumb-tip TT of a user.

The first cast portion 110 may comprise:
a cast proximal portion 110PP for enclosing the lower forearm LFA, wrist WR and at least the metacarpal portion MP of the hand of the user; and
one or both of:
   a finger distal portion 110FDP that is releasably connectable to the cast proximal portion 110PP for enclosing at least the finger proximal phalanx bones FPP and the finger intermediate phalanx bones FIP of the user; and
   a thumb distal portion 110TDP that is releasably connectable to the cast proximal portion 110PP for enclosing at least the thumb distal phalanx bone TDP of the user.

In a closed configuration, the first cast portion 110 and second cast portion 112 may abut along part or all of at a radial join 130R and an ulnar join 130U that respectively extend along the radial side RS and the ulnar side US of at least the lower forearm LFA and wrist WR of a user.

The first cast portion 110 and second cast portion 112 may meet at a lateral join 130L that extends between the radial join 130R and the ulnar join 130U,
wherein one or more releasable hinge connectors 140H may be provided at the lateral join 130L to releasably connect together the first cast portion 110 and the second cast portion 112, each of the one or more releasable hinge connectors 140H comprising a first hinge part 140H1 and a cooperating second hinge part 140H2 being provided different ones of the first cast portion 110 and the second cast portion 112.

According to a third aspect, there is provided a cast 100 for supporting the lower forearm LFA, wrist WR and at least the metacarpal portion MP of a hand of a user,
comprising a releasably connectable first cast portion 110 and second cast portion 112 for cooperatively enclosing the lower forearm LFA, wrist WR and metacarpal portion MP of the hand,
wherein in a closed configuration, the first cast portion 110 and second cast portion 112 abut along part or all of a radial join 130R and an ulnar join 130U that respectively extend along the radial side RS and the ulnar side US of at least the lower forearm LFA and wrist WR of a user.

The first cast portion 110 may extend across both the planar face (palmar face) PF and the dorsal face DF of the finger metacarpophalangeal joints FMJ, the finger proximal phalanx bones FPP, the finger intermediate phalanx bones FIP and the finger distal phalanx bones FDP of the hand of the user, and
the first cast portion 110 and second cast portion 112 may meet at (e.g. abut along part or all of) a lateral join 130L that extends between the radial join 130R and the ulnar join 130U.

One or more releasable hinge connectors 140H may be provided at the lateral join 130L to releasably connect together the first cast portion 110 and the second cast portion 112, each of the one or more releasable hinge connectors 140H comprising a first hinge part 140H1 and a cooperating second hinge part 140H2 being provided different ones of the first cast portion 110 and the second cast portion 112.

In each of the one or more releasable hinge connectors 140H:
the first hinge part 140H1 may comprise a projecting hinge tongue 140HT for engaging into the second hinge part 140H2; and
the second hinge part 140H2 may comprise a tapered hinge groove 140HG with an opening for receiving the projecting hinge tongue 140HT of the first hinge part 140H1, in which the tapered hinge groove 140HG increases in width 140HW away from the opening.

When the first cast portion 110 and the second cast portion 112 are in a connected configuration, the first hinge part 140H1 may abut the second hinge part 140H2 in an inner part of each releasable hinge connector 140H that is proximate the interior of the cast 100, and the first hinge part 140H1 may be spaced apart from the second hinge part 140H2 by a hinging gap 146 in an outer part of each releasable hinge connector 140H that is remote from the interior of the cast 100.

The cast may comprise an anti-pinch gap 130G along part of one or both of the radial join 130R and the ulnar join 130U, wherein the first cast portion 110 and the second cast portion 112 are configured to remain spaced apart along the anti-pinch gap 130G, when the first cast portion 110 and the second cast portion 112 are in the closed configuration.

The cast may comprise a comfort region 114 that have enhanced flexibility relative to the part of the cast 100 surrounding the comfort region 114.

One or more releasable alignment connectors 140A may be provided at each of the radial join 130R and the ulnar join 130U to releasably connect together the first cast portion 110 and the second cast portion 112, and
wherein each of the releasable alignment connectors 140A may comprise a first alignment connector part 140A1 having a projecting alignment tongue 140AT, and a complementary second alignment connector part 140A2 having an alignment groove 140AG, the first alignment connector part 140A1 and the second alignment connector part 140A2 being provided on different ones of the first cast portion 110 and the second cast portion 112.

In each of the one or more releasable alignment connectors 140A, the first alignment connector part 140A1 may comprise a projecting alignment tongue 140AT for engaging into the second side connector part 140A2, and the second alignment connector part 140A2 may comprise a tapered alignment groove 140AG with an opening for receiving the projecting alignment tongue 140AT of the first alignment connector part 140A1, in which the tapered alignment groove 140AG narrows in width away from the opening.

Each releasable hinge connector 140H or releasable alignment connector 140A may comprise a circumferential connector groove 142 for receiving a connection band 144 for releasably biasing together parts 140H1, 140H2, 140A1, 140A2 of each releasable hinge connector 140H or releasable alignment connector 140A.

The circumferential connector groove 142 may comprise a first region with a first depth 142D1 and a second region with a shallower second depth 142D2.

In the first region with the first depth 142D1, the circumferential connector groove 142 may have a concave cross-sectional shape with a maximal width MW that is greater than the width of the opening OW for receiving and retaining the connection band 144 within the circumferential connector groove 142 in the first region, when disconnected from the second region.

The distal end 100DE of the cast 100 may extend to the fingertips FT and thumb-tip TT of a user and the cast 100 may be provided with fingertip apertures 150F and a thumb-tip aperture 150T respectively adjacent the finger tips FT and the thumb-tip TT of the user.

In the closed configuration, the first cast portion 110 and the second cast portion 112 may extend through a respective first angle A1 and second angle A2 around each of the lower forearm LFA and wrist WR of the user, with respect to the centre of the cast 100 at the lower forearm LFA and wrist WR, wherein one of the first angle A1 and second angle A2 is 55% to 70% of 360 degrees.

A second hinge part 140H2 or a second alignment connector part 140A2 may comprise a sighting hole 148 for viewing a projecting hinge tongue 140HT or projecting alignment tongue 140AT within the hinge groove 140HG or alignment groove 140AG from perpendicular to the exterior surface of the cast 100.

A drainage hole 149 may be provided, extending from the tapered hinge groove 140HG or tapered alignment groove 140AG, to an exterior surface of the second hinge part 140H2 or second alignment connector part 140A2, extending parallel to an insertion direction ID of a complementary projecting hinge tongue 140HT or projecting alignment tongue 140AT.

The radial join 130R may extend at least partially along the proximal side and the distal side of the thumb TH of a user.

The cast 100 may comprise a thumb connection band 154A, 154B for connecting together the first cast portion 110 and the second cast portion 112 at the thumb TH of the user, and
a thumb band retainer 155A, 155B for retaining the thumb connection band 154A, 154B on the first cast portion 110 or the second cast portion 112 for retaining the thumb connection band 154A, 154B when the thumb connection band 154A, 154B is disconnected from the other of the first cast portion 110 and the second cast portion 112.

The first cast portion 110 or the second cast portion 112 may comprise a circumferential groove 152 extending around the thumb TH for receiving the thumb connection band 154A in the disconnected configuration, and
a thumb band lug 157A for engaging the thumb connection band 154A onto, to connect together the first cast portion 110 and the second cast portion 112 at the thumb TH of the user in the connected configuration.

The second cast portion 112 or first cast portion 110 may comprise a thumb connection band 154B for stretching between the thumb band retainer 155B and a thumb lug 157B of the first cast portion 110 or the second cast portion 112, to extend across the other of the first cast portion 110 or the second cast portion 112 in the connected configuration.

The cast 100 may have a first layer thickness T1 for covering the lower forearm LFA and wrist WR, and a thinner second layer thickness T2 for covering at least the finger metacarpophalangeal joints FMJ, wherein second layer thickness T2 is at least 15% less at the finger metacarpophalangeal joints FMJ.

Centrally along the planar face PF the cast 100 may have a third layer thickness T3 and centrally along the dorsal face DF the cast 100 may have a fourth layer thickness T4, wherein the fourth layer thickness T4 is at least 15% less than the third layer thickness T3.

At the radial join 130R and the ulnar join 130U, the first cast portion 110 and the second cast portion 112 may have a first curved edge C1 where the cast 100 has a first layer thickness T1 and a second curved edge C2 where the cast 100 has a second layer thickness T2, wherein the second layer thickness T2 is at least 15% less than the first layer thickness T1, wherein the radius of curvature of the second curved edge C2 is smaller than the radius of curvature of the first curved edge C1, and the radialjoin 130R and the ulnar join 130U have a mating face width 162 of at least 3 mm.

At the radial join 130R and the ulnar join 130U the first cast portion 110 and the second cast portion 112 may have curved edge C, C1, C2 having a radius of curvature of at least 0.5 mm.

The first cast portion 110 and the second cast portion 112 may extend across both the planar face PF and the dorsal face DF of the finger metacarpophalangeal joints FMJ to a distal end 100DE that is at the finger proximal phalanx bones FPP or beyond, and
adjacent at least the finger proximal phalanx bones FPP, the interior surface of the cast 100 is provided with ridges 160 projecting inwardly between at least the index finger F1, middle finger F2 and ring finger F3 of a user, wherein the minimal gap G1 between opposed ridges 160 aligned between the index finger F1 and the middle finger F2, and the minimal gap G3 between the opposed ridges 160 aligned between the middle finger F2 and the ring finger F3 are each at least 20% less than the maximal gap G2 between the opposed internal faces of the cast 100 aligned with the middle finger F2.

The first cast portion 110 may extend along the planar face (palmar face) PF of the lower forearm LFA, wrist WR, and the second cast portion 112 may extend along the dorsal face DF of the lower forearm LFA, wrist WR.

The first cast portion 110 may extend along the dorsal face DF of the lower forearm LFA, wrist WR, and the second cast portion 112 may extend along the planar face (palmar face) PF of the lower forearm LFA, wrist WR.

The cast 100 may extend longitudinally to a distal end 100DE at the finger proximal phalanx bones FPP of a user, or
wherein the cast 100 may extend longitudinally to a distal end 100DE at the finger metacarpophalangeal joints FMJ of the user.

The distal end 100DE may comprise a bevelled edge 164 around the fingers FG that is bevelled inwardly toward the fingers FG of the user by an angle BEV of at least 30 degrees relative to the perpendicular to the adjacent internal surface of the cast 100.

The distal end 100DE may comprise a rounded beading 166 around the fingers FG having a radius of curvature that is greater than half the thickness of the adjacent cast 100.

The various embodiments described above represent individual features of the invention which can be applied generally to the cast of the invention. These features may be taken individually as preferred features or more than one of these preferred features may be combined with one another in any combination.

### DESCRIPTION OF THE DRAWINGS

Examples are further described hereinafter with reference to the accompanying drawings, in which:
- Figures 1A and 1B show a cast in a closed and partially opened configuration, respectively;
- Figures 1C and 1D respectively show a first cast portion and a second cast portion of the cast of Figures 1A and 1B;
- Figures 1E and 1F show the lower forearm, wrist and hand of a user, respectively viewed from the radial side and the ulnar side;
- Figure 1G shows a further cast in the closed configuration;
- Figures 2A to 2G show a releasable alignment connector;
- Figures 3A to 3F show a releasable hinge connectors;
- Figures 4A to 4C show casts of different lengths;
- Figures 5A and 5B show casts having removable finger and thumb covers;
- Figure 6 shows the lower arm of a user with involuntarily contracted muscles in the wrist and fingers;
- Figures 7A to 7G show steps in the use of a further cast;
- Figure 8 shows part of a releasable alignment connector having a drainage hole;
- Figures 9A to 10B show thumb connections for a cast;
- Figures 11A to 11C show a cast having bevelled edges;
- Figures 11D to 11E show a cast having edges with enlarged rounded beading;
- Figures 12A and 12B show a cast having fingertip apertures and a thumb tip aperture respectively;
- Figure 13A shows a cut-away view of a cast having a non-uniform layer thickness;
- Figures 13B and 13C show cut-away views of a further cast having different planar and dorsal layer thickness;
- Figures 14A to 14C show cross-sectional views through a cast having curved edges at the radial join and the ulnar join;
- Figures 15A to 15C show a perspective view and cut-away views of a cast having a first cast portion and a second cast portion that extend around the lower forearm and wrist by unequal extents;
- Figures 16A and 16B show casts having ridges projecting inwardly between the fingers; and
- Figures 17A to 17D shows a further cast in the closed configuration.

### DETAILED DESCRIPTION

In the described examples, like features have been identified with like numerals.

The present application concerns improvements to medical casts to enhance the ease of fitting and removal, facilitate use by patients with involuntarily contracted wrist and hand muscles (e.g. patients who have experienced a stroke, or who have cerebral palsy), and to enhance performance and comfort in use.

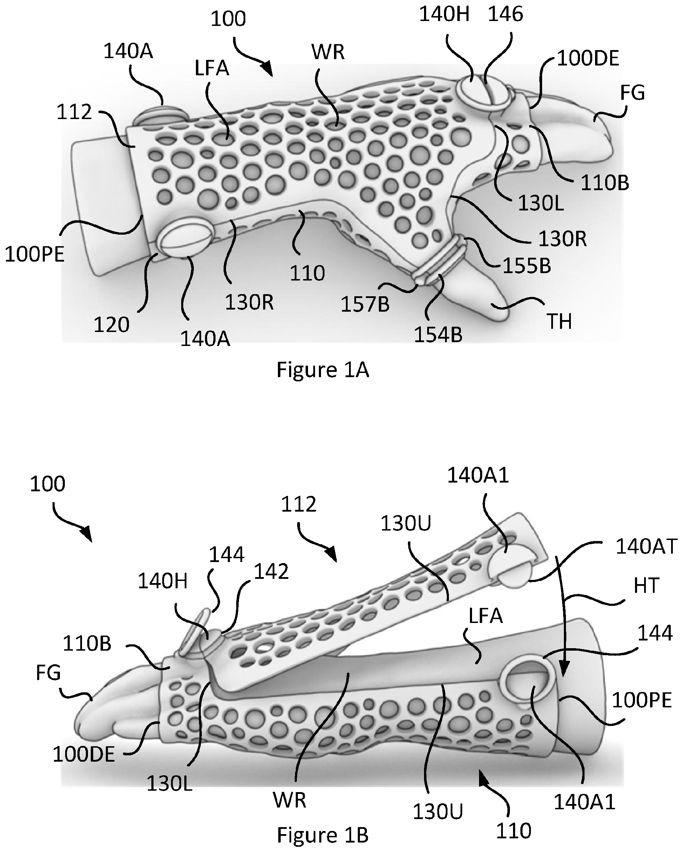

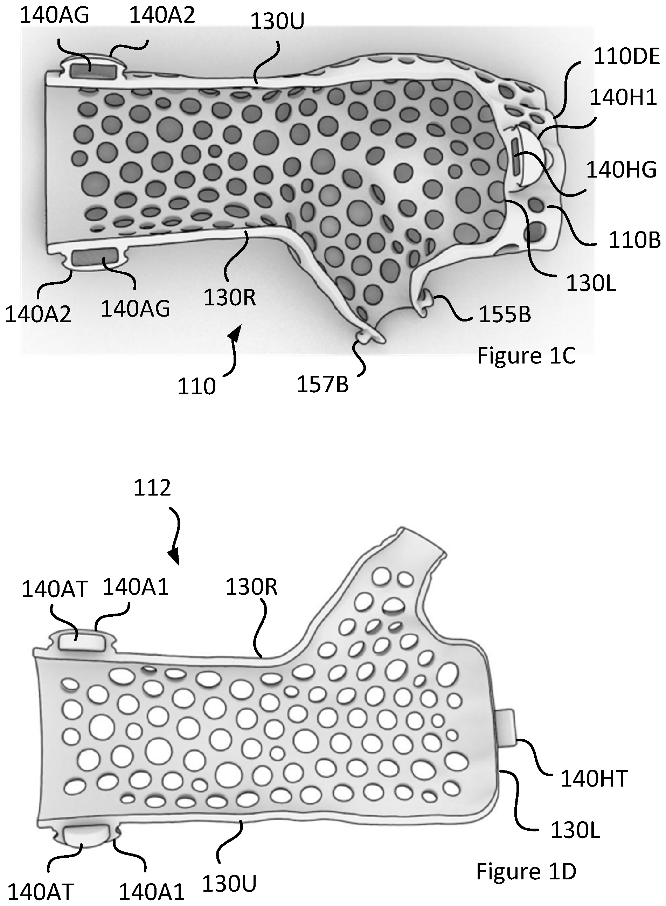

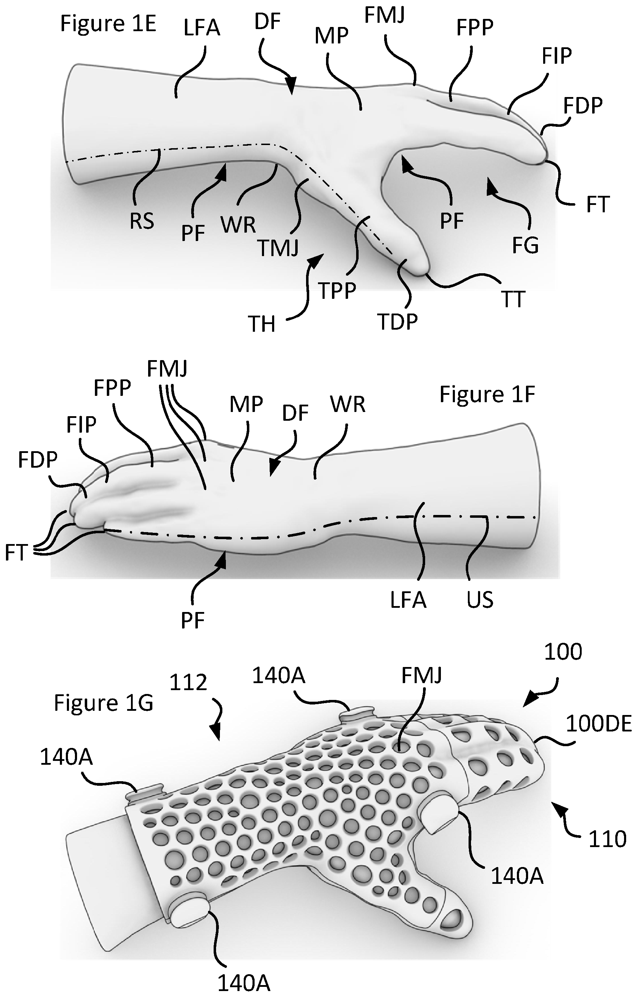

Figures 1A and 1B show a cast 100 for supporting the lower forearm LFA (the distal part of the upper limb, being distal from the elbow), wrist WR and at least the metacarpal portion MP of a hand of a user, Figures 1C and 1D show, separately, a first cast portion 110 and a second cast portion 112 of the cast 100. Figures 1Eand 1F show views of the lower forearm LFA, wrist WR and open hand of a user, respectively viewed from the radial side RS and ulnar side US.

The cast 100 is shaped to fit closely to the shape of the user's lower forearm LFA, wrist WR and hand.

When the dorsal face DF of the hand and forearm are rested on a horizontal surface, with the hand in an open configuration, in plan view, the "radial side" RS and "ulnar side" US are the opposed sides of the hand and forearm, corresponding with the sides on which the radial and ulnar bones are located in the forearm.

The cast 100 has a first cast portion 110 and a second cast portion 112, which releasably connect, in use, to enclose the lower forearm LFA, wrist WR and at least the metacarpal portion MP of the hand (the region of the hand in which the metacarpal bones are located). Each of the first cast portion 110 and the second cast portion 112 generally extends along one of the planar face (palmar face) PF and dorsal face DF of the lower forearm LFA, wrist WR and at least the metacarpal portion MP of the hand of the user.

The first cast portion 110 and second cast portion 112 abut along part or all of a radial join 130R that extends along or proximate the radial side RS of at least the lower forearm LFA and wrist WR of the user. The first cast portion 110 and second cast portion 112 also abut along part or all of an ulnar join 130U that extends along or proximate the ulnar side US of at least the lower forearm LFA and wrist WR of the user, i.e. on the opposed side of the lower forearm and wrist from the radial join 130R.

Being formed of two parts, the first cast portion 110 and the second cast portion 112, enables the cast 100 to be opened to receive the lower forearm LFA, wrist WR and at least the metacarpal portion MP of a hand of a user.

Being joined along the radial join 130R and the ulnar join 130U improves usability of the cast 100. The first cast portion 110 or the second cast portion 112 can be rested on a table, on user's upper legs, or on another supporting surface, whilst their lower forearm LFA, wrist WR and metacarpal portion MP of the hand are placed into that cast portion, with the weight of the lower arm of the user comfortably holding that cast portion in place. When the other cast portion 112, 110 is connected to the held cast portion 110, 112, the other cast portion may be connected by the user using only their other hand, enabling the cast 100 to be assembled without requiring the assistance of a carer or other third party.

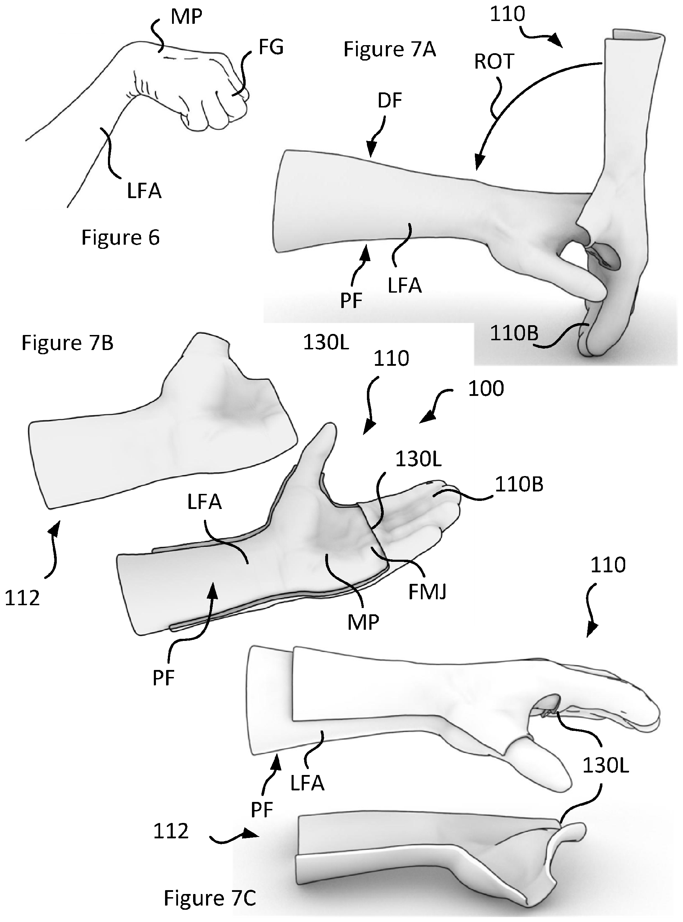

Having the first cast portion 110 and the second cast portion 112 joined by a radial join 130R and an ulnar join 130U may be beneficial to patients with hand muscles that are involuntarily contracted by clenching the planar face PF of the metacarpal portion MP of the hand (the palm) towards the wrist, and/or by closing the fingers FG towards the planar face PF of the metacarpal portion MP of the hand, for example, similarly to the clenched wrist and hand shown in Figure 6. By being joined along the radial join 130R and the ulnar join 130U, the planar face PF of the metacarpal portion MP of the hand may be placed gradually into the first cast portion 110, to enable clenched muscles of the wrist and hand to be gradually stretched by the patient under their own applied force and at a rate that is comfortable, causing reactionary forces to act upon the clenched muscles against their direction of involuntary clenching. The gradual stretching may happen before connecting the other cast portion 112, 110, or during the connection of the other cast portion 112, 110, or at both stages. Stretching the clenched muscles against their direction of involuntary clenching may provide a therapeutic benefit to the user.

The radial join 130R and the ulnar join 130U may extend along the cast 100 from the proximal end 100PE to the distal end 100DE (e.g. forming a generally tubular cast between the proximal end 100PE and the distal end 100DE at the fingers FG, with additional provision for the thumb TH).

Where the first cast portion 110 predominantly extends along the dorsal face DF or planar face PF of the lower forearm LFA, wrist WR and at least the metacarpal portion MP of a hand, the first cast portion 110 may also comprise a further portion that extends around the opposed face PF, DF of at least part of the hand. The first cast portion 110 and the second cast portion 112 may meet at (e.g. abut along part or all of) a lateral join 130L that extends from the radial join 130R and the ulnar join 130U.

In the example shown in Figures 1A to 1D, the first cast portion 110 is predominantly provided on the planar face PF of the lower forearm LFA, wrist WR and at least the metacarpal portion MP of a hand, along which it extends. Additionally, the first cast portion 110 comprises a band 110B that extends across the dorsal face DF of the finger proximal phalanx bones FPP, with a lateral join 130L extending across the dorsal face DF between the radial join 130R and the ulnar join 130U. The lateral join 130L may be adjacent (at or proximate) the finger proximal phalanx bones FPP (e.g. the lateral join 130L may not extend across the finger metacarpophalangeal joints FMJ, reducing potential discomfort from the knuckles abrading against the lateral join 130L).

Alternatively, the lateral join 130L may extend between the radial join 130R and the ulnar join 130U adjacent (at or proximate) the finger intermediate phalanx bones FIP or the finger distal phalanx bones FDP.

Providing the lateral join 130L beyond the finger metacarpophalangeal joints FMJ may enable the hand to slide into the first cast portion 110 or the second cast portion 112 without needing to insert the finger metacarpophalangeal joints FMJ through a constriction, and without requiring the fingers to be opened flat at the finger metacarpophalangeal joints FMJ.

Elements described as being "adjacent" to one another are near to each other, next to each other, or touching each other.

The distal end 100DE of the cast 100 is remote from the elbow of the user, in use, and the proximate end 100PE of the cast 100 is closest to the elbow of the user, in use.

In use, releasable alignment connectors 140A connect together the first cast portion 110 and second cast portion 112, connecting across the radial join 130R and the ulnar join 130U, and optionally across the lateral join 130L.

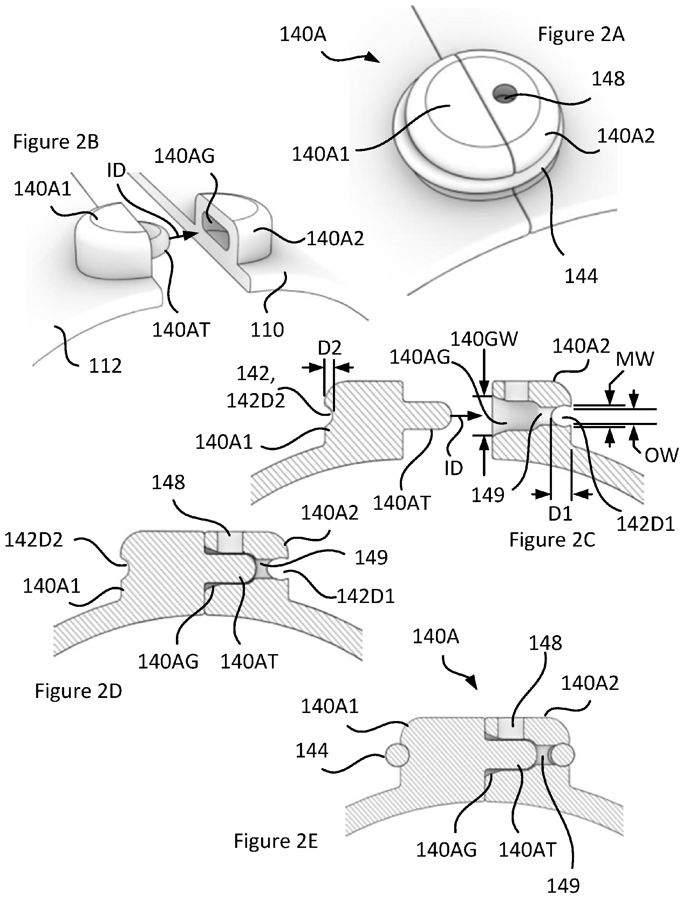

As shown in Figures 2A to 2E, each releasable alignment connector 140A has a first alignment connector part 140A1 and a second alignment connector part 140A2, which are complementary. The first alignment connector part 140A1 has a projecting alignment tongue 140AT. The second alignment connector part 140A2 has a complementary alignment groove 140AG for receiving the projecting alignment tongue 140AT when the first alignment connector part 140A1 and second alignment connector part 140A2 are engaged in the assembled cast 100.

In the illustrated cast 100, the first alignment connector part 140A1 that has the projecting alignment tongue 140AT has been provided on the second cast portion 112, and the second alignment connector part 140A2 that has the complementary alignment groove 140AG has been provided on the first cast portion 110. Alternatively, the first alignment connector part 140A1 and the second alignment connector part 140A2 may be provided on the first cast portion 110 and the second cast portion 112 respectively.

As is shown in Figure 2C, at least adjacent (at or proximate) the opening to the alignment groove 140AG, the alignment groove 140AG may have a groove width 140GW that is tapered, reducing in width way from the opening. The alignment tongue 140AT may have a constant width.

Alternatively, at least adjacent (at or proximate) the tip of the alignment tongue 140AT, the alignment tongue 140AT may be tapered, increasing in width away from the tip. In a further alternative, both the alignment groove 140AG and the alignment tongue 140AT may be tapered, as each separately described.

One or both of the tapered groove width 140GW adjacent the opening to the alignment groove 140AG and the tapered width of the alignment tongue 140AT adjacent the tip of the alignment tongue 140AT may simplify use of the cast 100, by guiding the projecting alignment tongue 140AT into the complementary alignment groove 140AG during assembly, which may facilitate use by patients with reduced dexterity.

The releasable alignment connector 140A has a connection feature, for maintaining the first alignment connector part 140A1 and the second alignment connector part 140A2 in an assembled configuration, as shown in Figures 2A to 2G.

The releasable alignment connector 140A may have an elastomeric connection band 144 (O-ring), which is under tension in use, for biasing the assembled first alignment connector part 140A1 and the second alignment connector part 140A2 to remain in the assembled configuration. The elastomeric connection band 144 may be a nitrile rubber or silicone rubber band. A elastomeric connection band 144 provides a low complexity mechanism for connecting together the first alignment connector part 140A1 and the second alignment connector part 140A2.

The releasable alignment connector 140A may have a circumferential connector groove 142 for receiving the elastomeric connection band 144, in the assembled configuration. The circumferential connector groove 142 provides a low complexity arrangement for retaining the elastomeric connection band 144 in place, when the releasable alignment connector 140A is in the connected configuration.

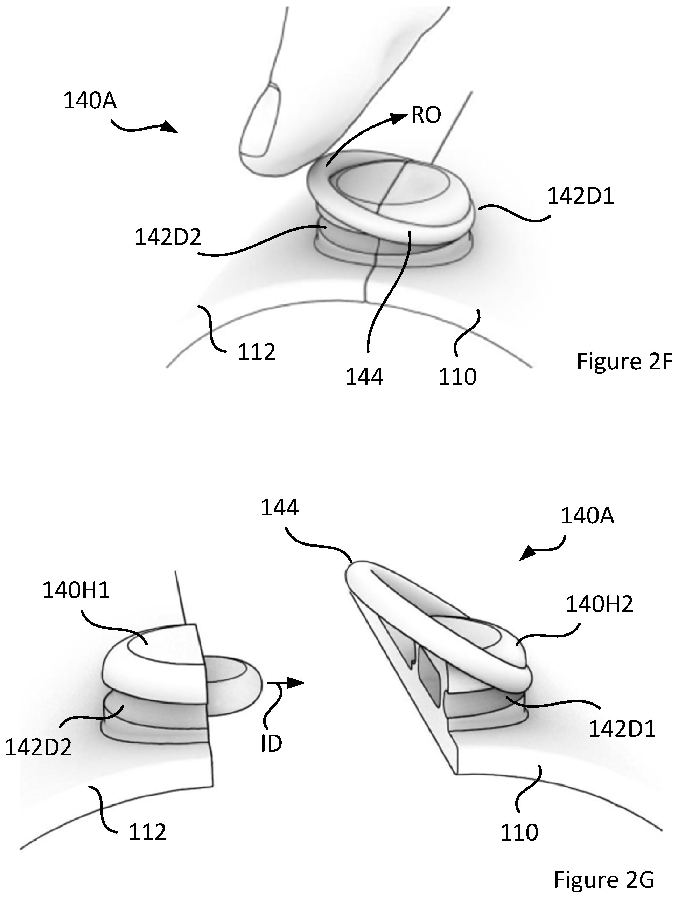

The circumferential connector groove 142 may have a non-uniform depth D1, D2 around the circumference of the releasable alignment connector 140A. The circumferential connector groove 142 may have a region 142D1 of first depth D1 for stably retaining the elastomeric connection band 144 in place, and a region 142D2 of shallower second depth D2. When the elastomeric connection band 144 is received into the region 142D2 of shallower second depth D2, a greater proportion of the elastomeric connection band 144 projects beyond the adjacent surface of the releasable alignment connector 140A than where the elastomeric connection band 144 is received into the region 142D1 of first depth D1. Provision of the region 142D1 of first depth D1 may enable the elastomeric connection band 144 to resist displacement from the circumferential connector groove 142 under the action of everyday minor impacts in use. as shown in Figure 2F, provision of the region 142D2 of shallower second depth D2 may enable the user to roll out RO a corresponding part of the elastomeric connection band 144 from the circumferential connector groove 142, for disconnecting the elastomeric connection band 144 from biasing together the first alignment connector part 140A1 and the second alignment connector part 140A2.

At least part of the region 142D1 of greater first depth D1 of the circumferential connector groove 142 may have an opening width OW that is narrower than the maximum width MW of the circumferential connector groove 142, as shown in Figure 2C, and narrower than the elastomeric connection band 144. The narrower opening width OW may retain the elastomeric connection band 144 within that part of the circumferential connector groove 142, even when the elastomeric connection band 144 has been otherwise withdrawn from the circumferential connector groove 142, when disconnecting the elastomeric connection band 144 from biasing together the first alignment connector part 140A1 and the second alignment connector part 140A2, as shown in Figures 1B and 2G. Retaining the elastomeric connection band 144 within the part of the region 142D1 of greater first depth D1 may prevent the elastomeric connection band 144 from unintentionally becoming completely detached from the releasable alignment connector 140A, retaining the elastomeric connection band 144 ready in position for reconnection and preventing the elastomeric connection band from becoming lost.

As shown in Figure 1G, the first cast portion 110 and the second cast portion 112 may be solely connected together by releasable alignment connectors 140A. The releasable alignment connectors 140A may be provided only at the radial join 130R and the ulnar join 130U. Connecting the first cast portion 110 and the second cast portion 112 with only a single type of connector 140A may simplify manufacturing. Connecting the first cast portion 110 and the second cast portion 112 with only releasable alignment connectors 140A at the radial join 130R and the ulnar join 130U may simplify manufacturing.

As shown in Figure 1B, the cast 100 may have one or more releasable hinge connectors 140H, for connecting together the first cast portion 110 and the second cast portion 112 at a releasable hinge 140H, on the dorsal face DF of the hand.

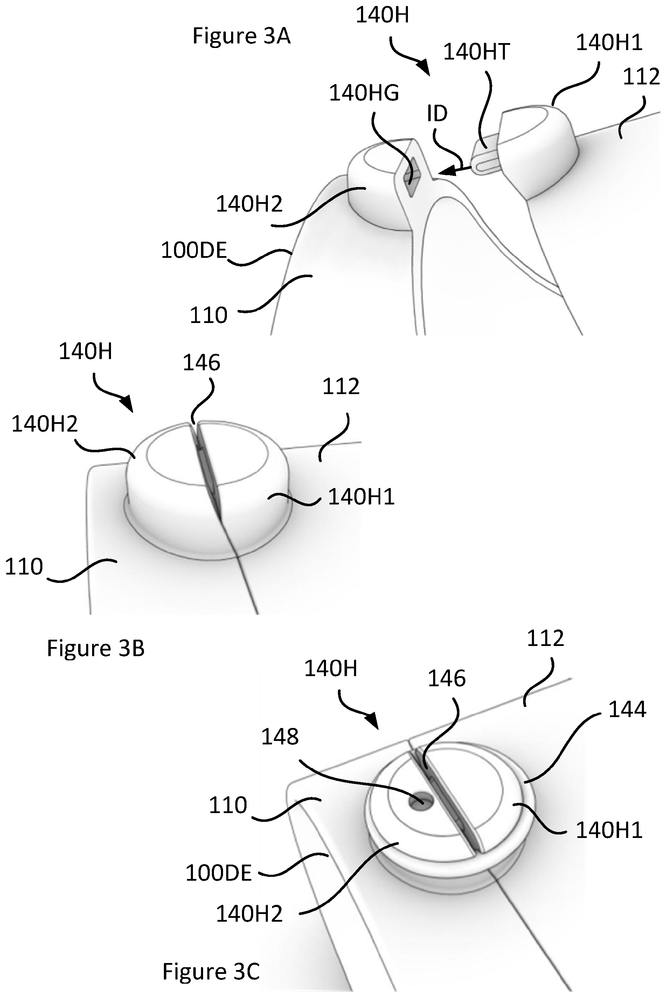

Each of the one or more releasable hinge connectors 140H has a first hinge part 140H1 and a complementary second hinge part 140H2 being provided on different ones of the first cast portion 110 and the second cast portion 112, as shown in Figures 3A to 3F.

The first hinge part 140H1 and the second hinge part 140H2 are brought together, with the first cast portion 110 and the second cast portion 112 angled apart in an open configuration, for example as shown in Figure 1B, before the first cast portion 110 and the second cast portion 112 are hinged together HT into the closed configuration, and the first cast portion 110 and the second cast portion 112 are secured in the closed configuration using further releasable connectors 140A (e.g. releasable alignment connectors 140A), as shown in Figure 1A. Connecting together the first cast portion 110 and the second cast portion 112 with one or more releasable hinge connectors 140H in a first connection step, before hinging together HT into a closed configuration, may enhance usability of the cast 100 by reducing the number of connectors 140H that need to be aligned during any single assembly step, and may facilitate alignment of the other releasable alignment connectors 140A when hinging together HT into the closed configuration.

The one or more releasable hinge connectors 140H may enable the first cast portion 110 and the second cast portion 112 to hinge (hinging together HT) through an angle of at least 10 degrees, e.g. 20 degrees.

One or more releasable hinge connectors 140H may be provided at the lateral join 130L and releasable alignment connectors 140A may be provided at the radial join 130R and the ulnar join 130U.

Providing only one releasable hinge connectors 140H at the lateral join 130L may enable transverse relative movement of the first cast portion 110 and the second cast portion 112 (movement transverse to the plane of relative movement for hinging together HT), as they are closed upon the lower forearm LFA, without disengagement of the first hinge part 140H1 and the second hinge part 140H2, which may enhance usability, by accommodating natural biomechanics of operation of the cast 100 by the opposite hand, and may enable deliberate movement out the plane of movement for hinging together HT to reduce a risk of pinching loose skin between the first cast portion 110 and the second cast portion 112.

Alternatively, one or more releasable hinge connectors 140H may be provided at one of the radial join 130R and the ulnar join 130U, with releasable alignment connectors 140A at the opposed ulnar join 130U or radial join 130R.

The first hinge part 140H1 has a projecting hinge tongue 140HT. The second hinge part 140H2 has a complementary hinge groove 140HG for receiving the projecting hinge tongue 140HT when the first hinge part 140H1 and second hinge part 140H2 become mechanically engaged, when assembling the cast 100.

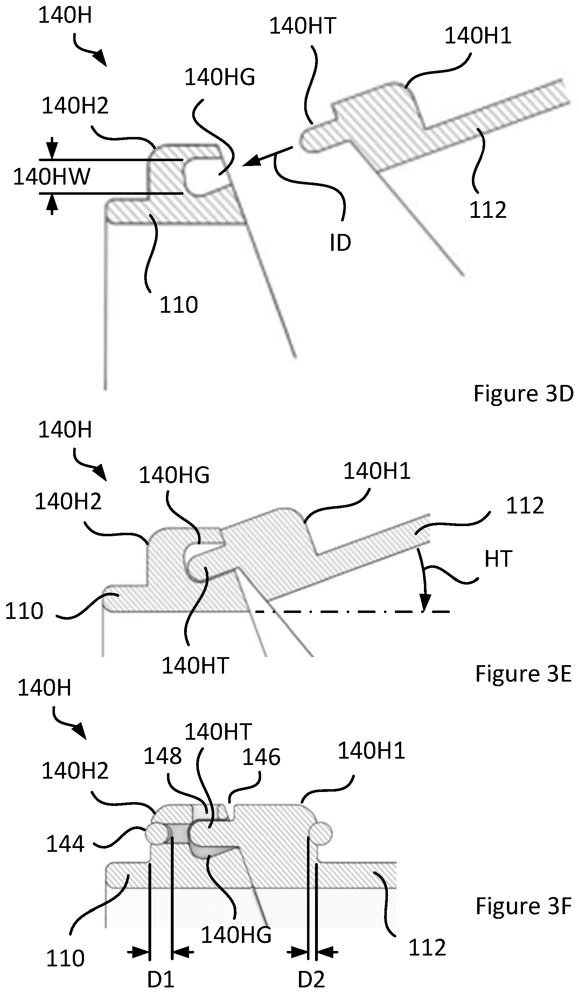

As is shown in Figure 3D, 3E and 1B, in use, the projecting hinge tongue 140HT is inserted ID into the complementary hinge groove 140HG in an initial engagement configuration. Then, the second cast portion 112 is hinged HT relative to the first cast portion 110, into the fully engaged configuration shown in Figures 3F and 1A, in which the first cast portion 110 and the second cast portion 112 are brought together along the radial join 130R and the ulnar join 130U.

The hinge groove 140HG may have a hinge groove width 140HW that is tapered, increasing in width way from the opening. The hinge tongue 140HT may have a constant width.

Alternatively, the hinge tongue 140HT may be tapered, increasing in width away from the tip. In a further alternative, both the hinge groove 140HG and the hinge tongue 140HT may be tapered as described.

As shown in Figures 1A, 3B, 3C and 3F, when the first cast portion 110 and the second cast portion 112 are in the closed configuration (closed at the radial join 130R and the ulnar join 130U), there may be a hinging gap 146 between the outer parts of the first hinge part 140H1 and the second hinge part 140H2, remote from the interior of the cast 100 (e.g. on the opposite side of the projecting hinge tongue 140HT from where the user's arm is received). Inner parts of the first hinge part 140H1 and the second hinge part 140H2, which are proximate the interior of the cast 100 (e.g. on the same side of the projecting hinge tongue 140HT from where the user's arm is received) may be in contact, when the first cast portion 110 and the second cast portion 112 are in the assembled configuration.

The provision of the hinging gap 146 facilitates cooperation between the first hinge part 140H1 and the second hinge part 140H2 of the releasable hinge connectors 140H without deformation, as they hinge together. Additionally, the provision of the hinging gap 146 may simplify assembly of the first cast portion 110 and the second cast portion 112, by enabling pivoting movement at the opening of the hinge tongue 140HT with little or no longitudinal movement of the first cast portion 110 relative to the second cast portion 112, facilitating the first cast portion 110 and the second cast portion 112 coming together into the closed configuration.

In the illustrated cast 100, the first hinge part 140H1 having the projecting hinge tongue 140HT has been provided on the second cast portion 112, and the second hinge part 140H2 having the complementary hinge groove 140HG has been provided on the first cast portion 110. Alternatively, the first hinge part 140H1 and the second hinge part 140H2 may be provided on the first cast portion 110 and the second cast portion 112 respectively.

As described for the releasable alignment connectors 140A, the one or more releasable hinge connectors 140H may (optionally) also have a connection feature (retention feature), for further securing the first hinge part 140H1 and the second hinge part 140H2 in an assembled configuration, as shown in Figures 1A, 1B, 3C and 3F.

The releasable hinge connectors 140H may have an elastomeric connection band 144 (O-ring, e.g. a nitrile rubber band or silicone rubber band), under tension in use, for biasing the first hinge part 140H1 and the second hinge part 140H2 into the assembled configuration. Additionally, the releasable hinge connectors 140H may have a circumferential connector groove 142 for receiving the elastomeric connection band 144, in the assembled configuration.

On each of the releasable alignment connectors 140A, the circumferential connector groove 142 may have a non-uniform depth D1, D2 around the circumference of the releasable alignment connector 140A, providing a region of greater depth D1 for stably retaining the elastomeric connection band 144 in place, and a region of shallower depth D2 for facilitating disconnection of the elastomeric connection band 144. Further, at least part of the region of greater first depth D1 of the circumferential connector groove 142 of the releasable hinge connectors 140H may have an opening width OW that is narrower than the maximum width MW of the circumferential connector groove 142, for retaining the disconnected elastomeric connection band 144, as shown in Figures 1B and 3F.

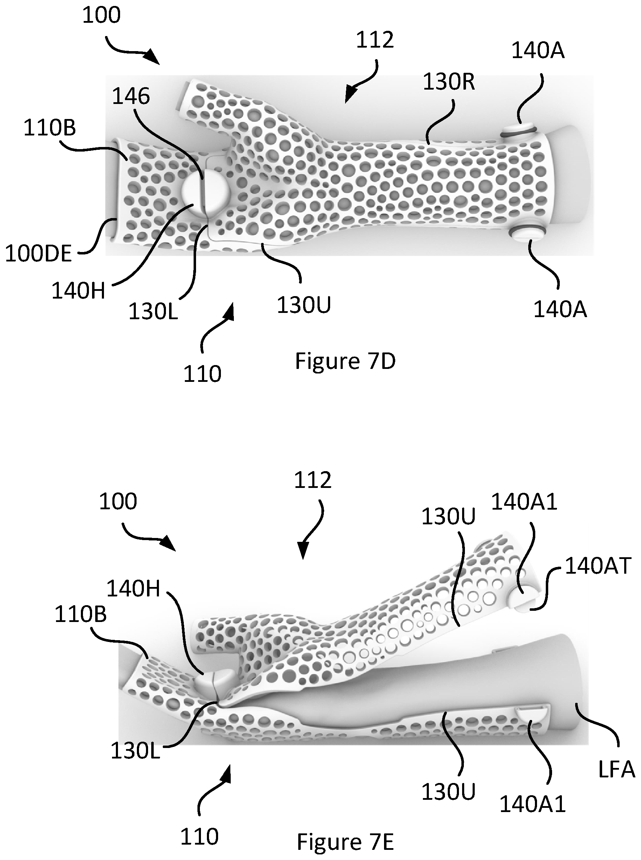

As shown in Figures 7D to 7G, the releasable hinge connectors 140H may be provided without a connection feature (e.g. the elastomeric connection band 144 shown in Figures 1A and 3F) for securing the first hinge part 140H1 and the second hinge part 140H2 in an assembled configuration. Providing the releasable hinge connectors 140H without a connection feature may enable reduced complexity manufacturing and use of the cast 100.

The releasable alignment connectors 140A may each have a sighting hole 148 for viewing a projecting alignment tongue 140AT that is received within the alignment groove 140AG, as shown in Figures 2A, 2C and 2E. Similarly, the one or more releasable hinge connectors 140H may each have a sighting hole 148 for viewing a projecting hinge tongue 140HT that is received within the hinge groove 140HG, as shown in Figures 3C and 3F. The sighting hole 148 may extend in through the second alignment connector part 140A2 or the second hinge part 140H2 approximately perpendicular to the exterior surface of the cast 100 (and approximately perpendicular to the tongue 140AT, 140HT.

Use of the sighting hole 148 to view the corresponding tongue can provide a user with a visual check to show that each releasable alignment connector 140A or releasable hinge connector 140H has been correctly assembled.

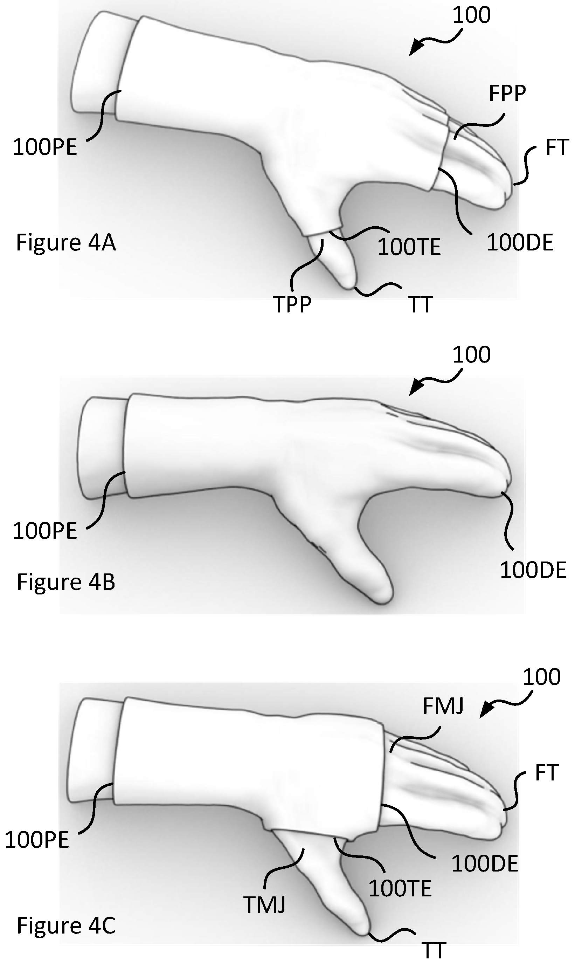

On the fingers FG of the user, the cast 100 (e.g. the first cast portion 110 and the second cast portion 112) may extend to a distal end 100DE at the finger proximal phalanx bones FPP, as shown in Figures 1A, 1B and 4A. The cast 100 may extend to a distal end 100DE at the finger intermediate phalanx bones FIP. As shown in Figure 4C, the cast 100 may extend to a distal end 100DE adjacent (at or proximate) the finger metacarpophalangeal joints FMJ of the user. A medium length or short cast 100 may enables the user to retain at least partial use of their fingers.

As shown in Figures 1G and 4B, the cast 100 may be a full length cast that extends to, and provides a substantially rigid enclosure around, a distal end 100DE at the finger tips FT of the user. A full length cast that includes coverage of the thumb TH provides support (and protection) for the whole hand.

Similarly to the distal end 100DE on the fingers FG, on the thumb TH of the user, the cast 100 may extend to a thumb end 100TE at the thumb proximal phalanx bone TPP or the thumb distal phalanx bone TDP, may stop adjacent (at or proximate) the thumb metacarpophalangeal joint TMJ, or the cast 100 may extend to the thumb tip TT.

As shown in Figures 1A and 1B, the cast 100 may extend from a proximal end 100PE in the lower forearm LFA. The proximal end 100PE may be at least 90mm (e.g. 90mm to 110mm) from the wrist WR, towards the elbow of the user, or at least one third of the distance from the wrist WR to the elbow (not shown).

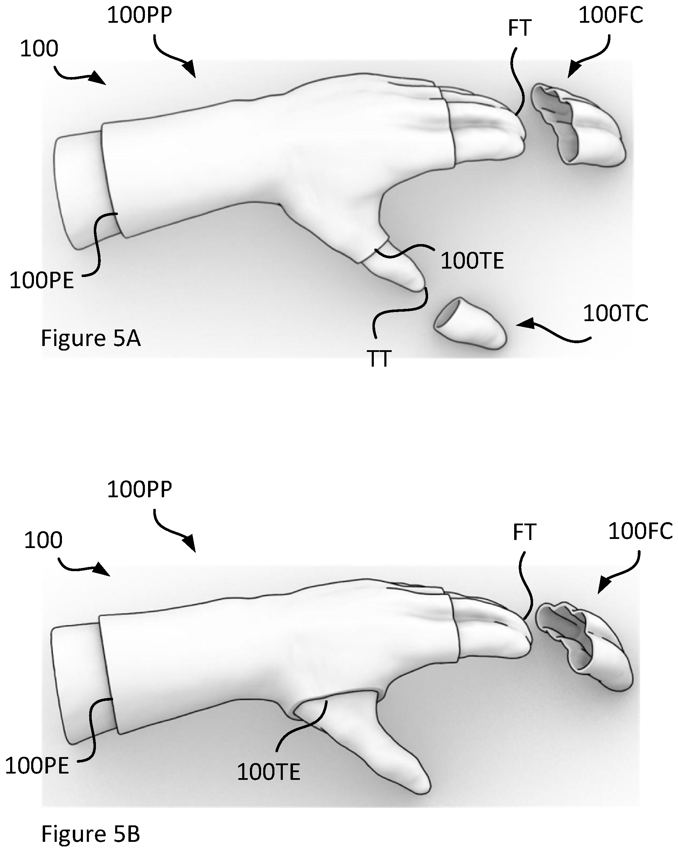

As shown in Figures 5A and 5B, the cast 100 may comprise a proximal portion 100PP that extends to the finger metacarpophalangeal joints FMJ, the finger proximal phalanx bones FPP, or the finger intermediate phalanx bones FIP (as discussed in relation to Figures 4A and 4C), and comprise a further releasably connectable finger cover 100FC that extends from the proximal portion 100PP to the finger tips FT, in use.

The releasably connectable finger cover 100FC enables the cast 100 to be converted between a full length cast, for example to provide maximum support and protection to the fingers whilst in bed, and enables the releasably connectable finger cover 100FC to be removed to provide the user with at least partial use of their fingers, for example whilst undertaking daytime tasks.

As shown in Figure 5A, the cast 100 may additionally comprise a releasably connectable thumb cover 100TC that extends from the proximal portion 100PP to the thumb tip TT, in use.

Figure 6 shows a hand in which the hand muscles are involuntarily contracted by clenching the planar face PF of the metacarpal portion MP of the hand (palm) towards the wrist, and by closing the fingers FG towards the planar face PF of the metacarpal portion MP of the hand.

Figures 7A to 7C show a further cast 100 in which the first cast portion 110 extends along the dorsal face DF of the lower forearm LFA, wrist WR and the hand, extending to the finger tips FT, in which the first cast portion 110 also comprises a planar portion, band 110B, that extends across the opposed planar face PF of at least part of the fingers FG (e.g. at least the proximal or the distal portions of the fingers FG), with a lateral join 130L extending between the radial join 130R and the ulnar join 130U. The lateral join 130L may be adjacent (at or proximate) the finger metacarpophalangeal joints FMJ.

The planar portion of first cast portion 110 that extends across the planar face PF, band 110B, may extend from the finger metacarpophalangeal joints FMJ to the finger tips FT (as shown in Figures 7A to 7C), from the finger proximal phalanx bones FPP to the fingertips FT, or from the finger intermediate phalanx bones FIP to the finger tips FT.

The planar portion of first cast portion 110 that extends across the planar face PF, band 110B, may extend from the finger metacarpophalangealjoints FMJ to the finger intermediate phalanx bones FIP or the finger proximal phalanx bones FPP, as shown in Figures 7D to 7G.

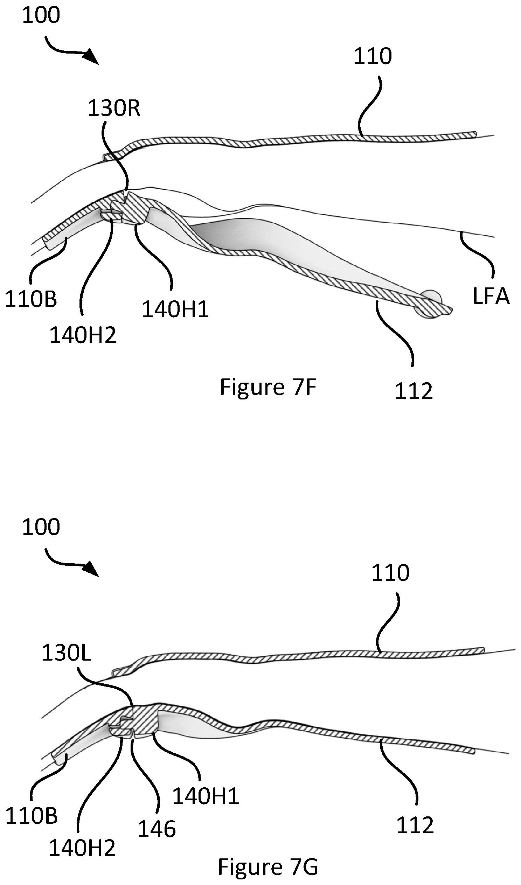

Providing a first cast portion 110 with a band 110B that extends along at least part of the planar face PF of the fingers (e.g. to the finger tips FT) may assist use of the cast 100 by patients with involuntarily contracted wrists and hands, as shown in Figure 6. In use, the finger tips FT may be inserted into the correspondingly shaped distal part of the first cast portion 110, as shown in Figure 7A. Then, the first cast portion 110 may be gradually rotated ROT relative to the lower forearm LFA by the patient under their own applied force to bring the opposite proximal end of the first cast portion 110 to the lower forearm LFA. That movement gradually stretches the clenched muscles against their direction of involuntary clenching, opening the involuntarily clenched fingers FG and/or wrist WR as shown in Figures 7B, 7C, 7E and 7F. Then, the second cast portion 112 may be releasably connected to the first cast portion 110 by releasable connectors.

As shown in Figures 7D to 7G, the cast 100 may have one or more releasable hinge connectors 140H, for connecting together the first cast portion 110 and the second cast portion 112 at a releasable hinge 140H, on the planar face PF of the hand. Figures 7E and 7F show the hinge parts 140H1, 140H2 of the releasable hinge connectors 140H engaged, before the cast portions 110, 112 are closed together around the lower forearm LFA, as shown in Figures 7D and 7G.

The releasable connectors may be releasable alignment connectors 140A, and any releasable hinge connector(s) 140H, similar to those described previously. Stretching the clenched muscles of the hand and/or wrist against their direction of involuntary clenching may provide a therapeutic benefit to the user.

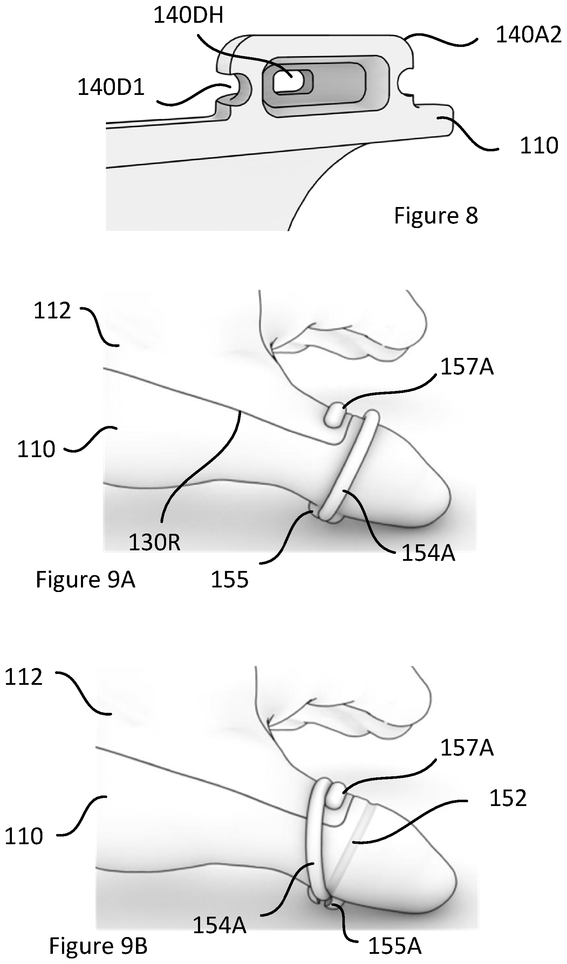

Some or all of the second alignment connector parts 140A2 may each have a drainage hole 140DH coupling from the alignment groove 140AG to the exterior of the second alignment connector part 140A2, as shown in Figure 8. Similarly, the second hinge part 140H2 may be provided with a drainage hole 140DH coupling from the hinge groove 140HG of to the exterior of the second hinge part 140H2. The draining hole 140DH may prevent the collection of residue within the alignment groove 140AG or hinge groove 140HG during manufacturing by 3D printing.

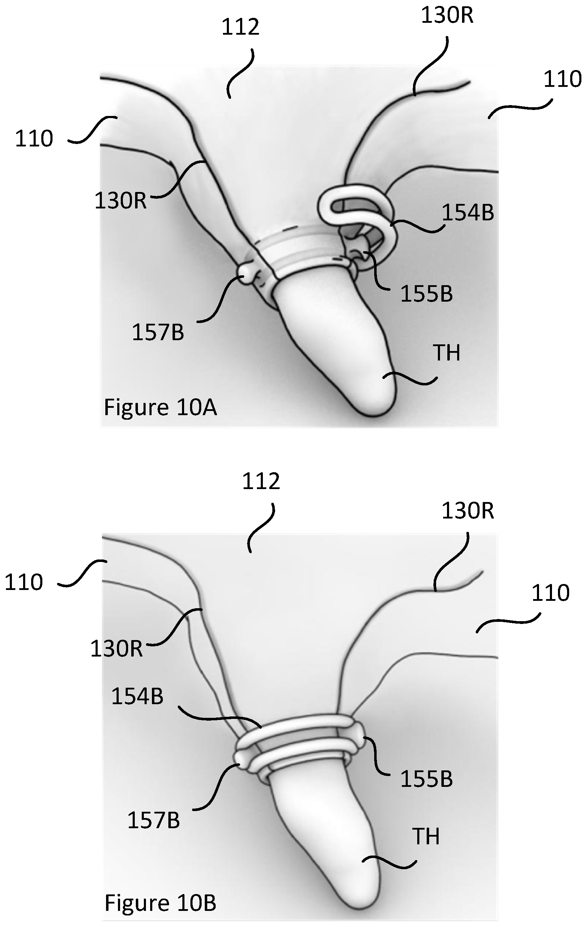

The first cast portion 110 and the second cast portion 112 may both extend along at least part of the thumb TH (e.g. both extending to at least the thumb proximal bone TPP of the user), abutting at the radial join 130R that extends along opposed sides of the thumb TH. As shown in Figures 9A to 10B, a thumb connection band 154A, 154B may be provided for connecting together the first cast portion 110 and the second cast portion 112 at the thumb TH of the user. Connecting together the first cast portion 110 and the second cast portion 112 at the thumb TH may reduce the risk of the first cast portion 110 and the second cast portion 112 becoming separated under the action of everyday impacts in use, and in reaction to involuntary contraction of muscles in the thumb TH.

The first cast portion 110 or the second cast portion 112 may have a thumb band retainer 155A, 155B for retaining the thumb connection band 154A, 154B, when the thumb connection band 154A, 154B is disconnected from the other of the first cast portion 110 and the second cast portion 112.

As shown in Figures 9A and 9B, the first cast portion 110 or the second cast portion 112 may have a circumferential groove 152 extending around the thumb TH for receiving the thumb connection band 154A (also extending around the thumb TH) in the disconnected configuration.

The opposed second cast portion 112 or first cast portion 110 may have a thumb band lug 157A for engaging the thumb connection band 154A onto, to connect together the first cast portion 110 and the second cast portion 112 at the thumb TH of the user in the connected configuration.

Extending the thumb connection band 154A around the thumb TH may enable the thumb connection band 154A to be moved between the connected and disconnected configurations by a rolling motion, which may be facilitate connection and disconnection by a user with limited manual dexterity.

As shown in Figures 10A and 10B, the first cast portion 110 or the second cast portion 112 may be connected together by stretching the thumb connection band 154B between two parts of the first cast portion 110 or the second cast portion 112 (e.g. from the thumb band retainer 155B and the thumb lug 157B), extending across the other of the first cast portion 110 or the second cast portion 112 (e.g., which may enable the first cast portion 110 and the second cast portion 112 to be secured together where the cast 100 does not extend to the thumb tip TT).

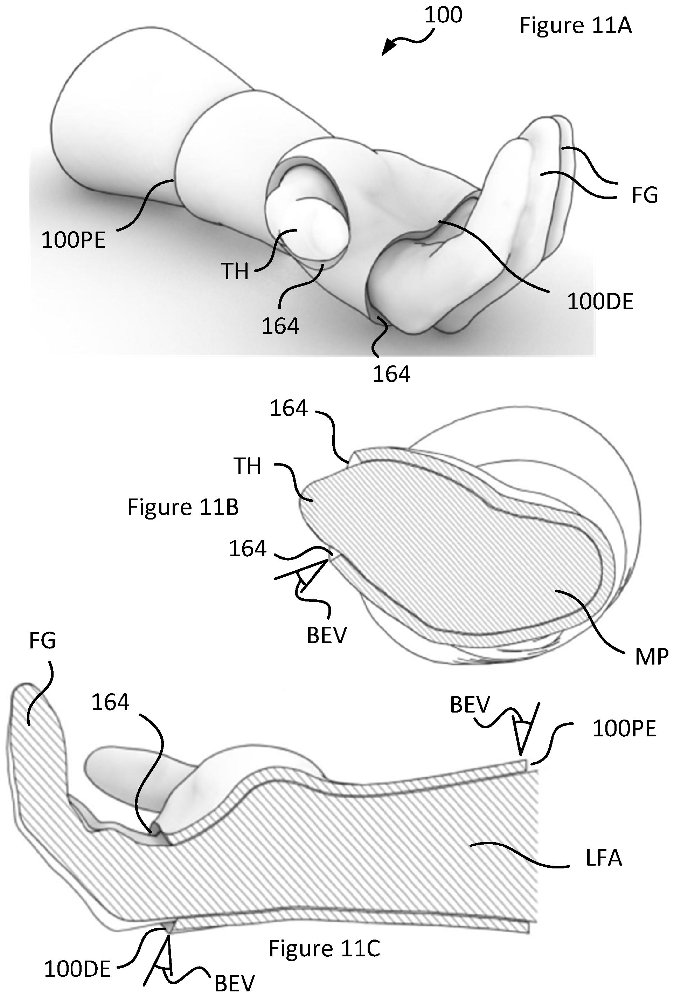

As shown in Figures 11A to 11C, around the fingers FG, the distal end 100DE of the cast 100 may have a bevelled edge 164 that is bevelled inwardly toward the fingers FG by an angle BEV of at least 30 degrees (e.g. 45 degrees) relative to the perpendicular to the adjacent internal surface of the cast 100. The cast 100 may also comprise a bevelled edge 164 around the thumb TH. The cast 100 may also comprise a bevelled edge 164 around the proximal end 100PE.

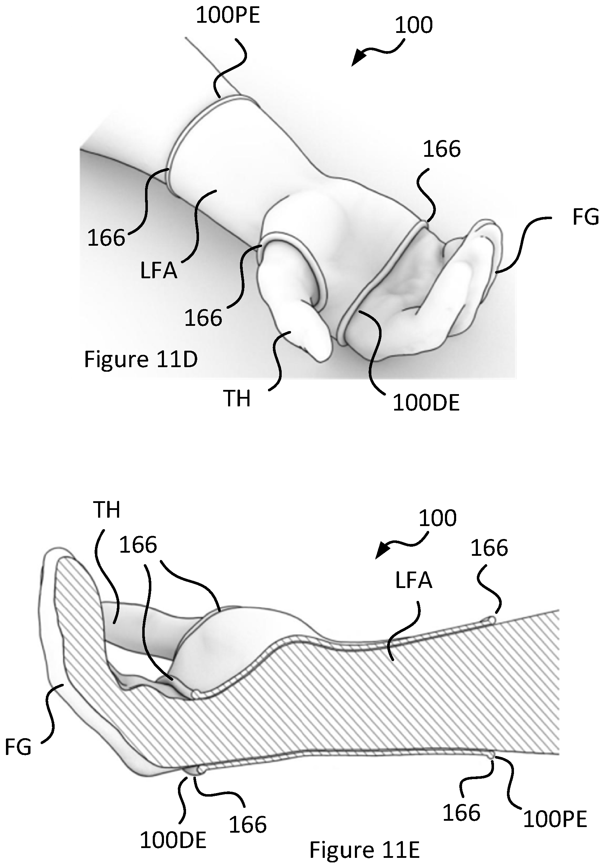

Alternatively, as shown in Figures 11D and 11E, around the fingers FG, the distal end 100DE of the cast 100 may have an enlarged rounded beading 166 having a radius of curvature that is greater than half the thickness of the adjacent cast 100 (e.g. at least 50% greater). The cast 100 may also comprise an enlarged rounded beading 166 around the thumb TH. The cast 100 may also comprise an enlarged rounded beading 166 around the proximal end 100PE.

Providing a bevelled edge 164 or an enlarged rounded beading 166 around some or all of the edges of the cast 100 may reduce the risk of skin abrasion in use and enhance user comfort.

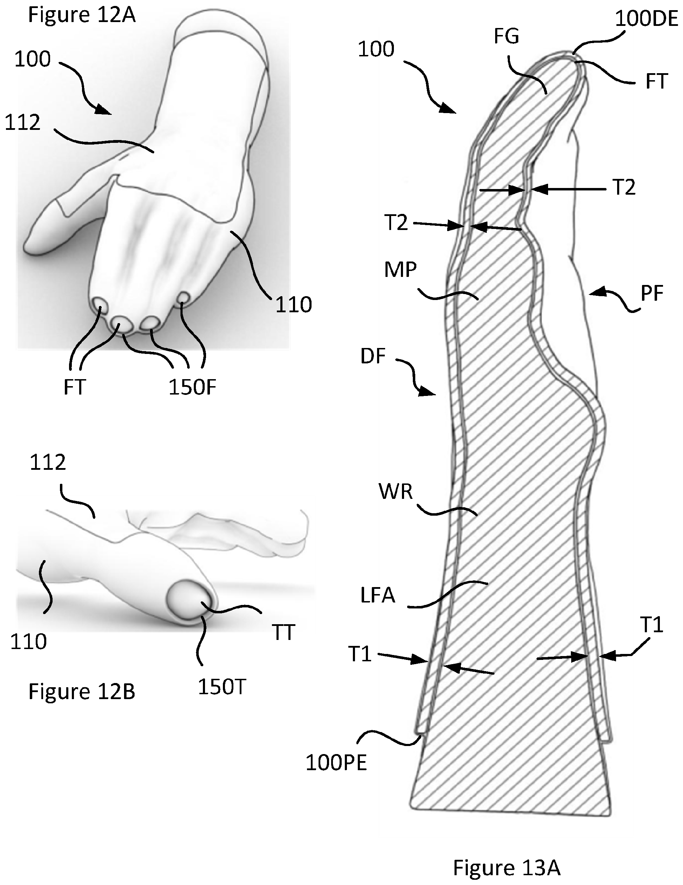

As shown in Figure 12A, when the distal end 100DE of the cast 100 extends to the fingertips FT the cast 100 may be provided with fingertip apertures 150F adjacent the finger tips FT. As shown in Figure 12B, when the cast 100 extends to the thumb tip TT, the cast 100 may be provided with a thumb tip aperture 150T adjacent the thumb tip TT.

Providing one or more of the fingertip apertures 150F may enhance user comfort, by reducing the risk of end-on contact between respective finger nails and the interior surface of the distal end 100DE of the cast. Providing external access to the fingertips FT through the fingertip apertures 150F may enable nail care to be provided (e.g. cutting finger nails) without removing the cast. Similarly, providing a thumb tip aperture 150T may enhance user comfort, by reducing the risk of end-on contact between thumb nail and the interior surface of the cast 100 adjacent the thumb tip TT, and enable nail care to the thumb nail. Further, providing fingertip apertures 150F and any a thumb aperture 150T may enable the user to experience tactile sensations through the fingertips FT and thumb tip TT, which may reduce the risk of the user experiencing a loss of tactile sensitivity during extended use (or lessen the time for which it may be necessary to remove the cast 100 for this purpose).

As shown in Figure 13A (and similarly in Figures 13A to 14C), the cast 100 may have a greater layer thickness proximate the proximate end 100PE of the cast 100 than at the distal end 100DE (excluding where connection features are provided for connecting together, e.g. releasable alignment connectors 140A and releasable hinge connectors 140H. The cast 100 may have a first layer thickness T1 (e.g. 4mm) where it covers the lower forearm LFA and wrist WR, and a thinner second layer thickness T2 (e.g. 2mm) for covering at least the finger metacarpophalangeal joints FMJ, and where the cast 100 extends to some or all of them, covering the finger proximal phalanx bones FPP, the finger intermediate phalanx bones FIP and the finger distal phalanx bones FDP. In the direction towards the fingertips FT, the reduction in thickness of the cast 100 from the first layer thickness T1 may commence immediately beyond the wrist WR, from the middle of the metacarpal portion MP of the hand (e.g. just beyond the thumb metacarpophalangeal joint TMJ) or adjacent the finger metacarpophalangeal joints FMJ. The second layer thickness T2 may be at least 15% less at the finger metacarpophalangeal joints FMJ, e.g. at least 25% less, or at least 33% less.

Maintaining the greater thickness T1 in the proximal part of the cast 100 maintains the maximum strength where the cast 100 may experience greater forces in use. Tapering to a smaller layer thickness T2 in the distal part of the cast 100 reduces the weight of the cast 100 (e.g. may reduce the weight by 15% compared with a uniform thickness), and disproportionately reduces the moment of inertia of the cast 100 with respect to rotation about the elbow or centre of the torso, both of which enhance user comfort, for example when turning around or when pivoting the lower arm at the elbow in ordinary use. Additionally, a smaller layer thickness T2 may increase flexibility of the cast 100 in the distal part of the cast 100, which may increase user comfort.

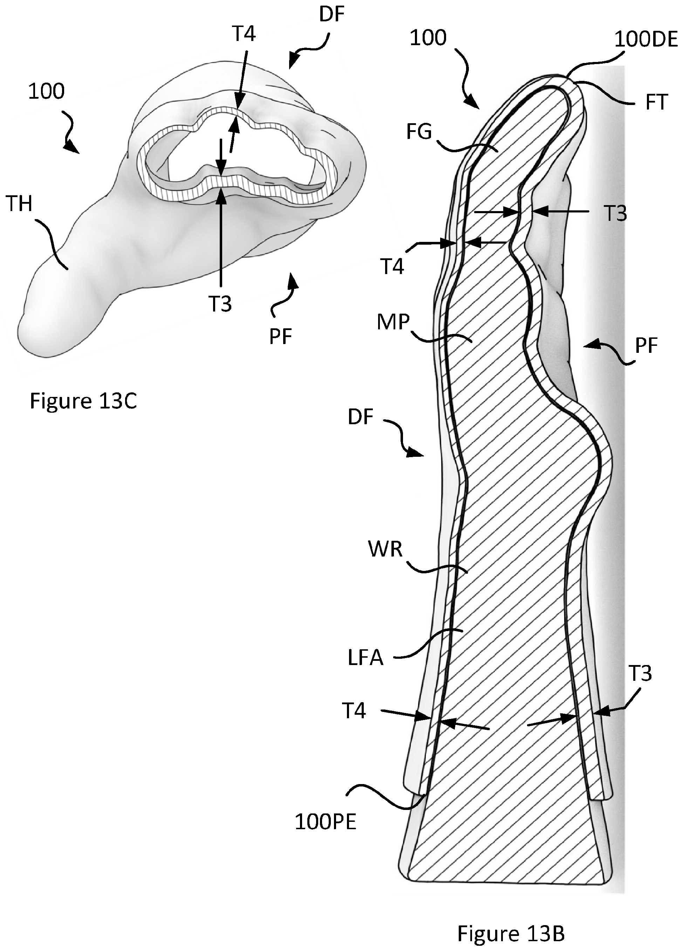

As shown in Figures 13B and 13C, centrally along the planar face PF, the cast 100 may have a third layer thickness T3 (e.g. 4mm), and centrally along the dorsal face DF the cast 100 may have a fourth layer thickness T4 (e.g. 2mm). The fourth layer thickness T4 may be at least 15% less than the third layer thickness T3 (e.g. at least 25% less). Extending centrally along each of the planar face PF and the dorsal face DF is substantially equidistant from the radial join 130R and the ulnar join 130U, in the lower forearm LFA, wrist WR and at least the metacarpal portion MP of the hand (aside from where the radial join 130R extends on the thumb TH).

Where a user has involuntarily contracted wrist and hand muscles, in use the hand may apply greater forces to the planar face PF of the cast 100 than to the dorsal face DF. Further, in use, the planar face PF may experience more impacts than the distal face DF. Providing a greater thickness T3 on the planar face PF of the cast 100 provides greater strength at the planar face PF to better resist greater forces and impacts to which the planar face PF may be exposed in use.

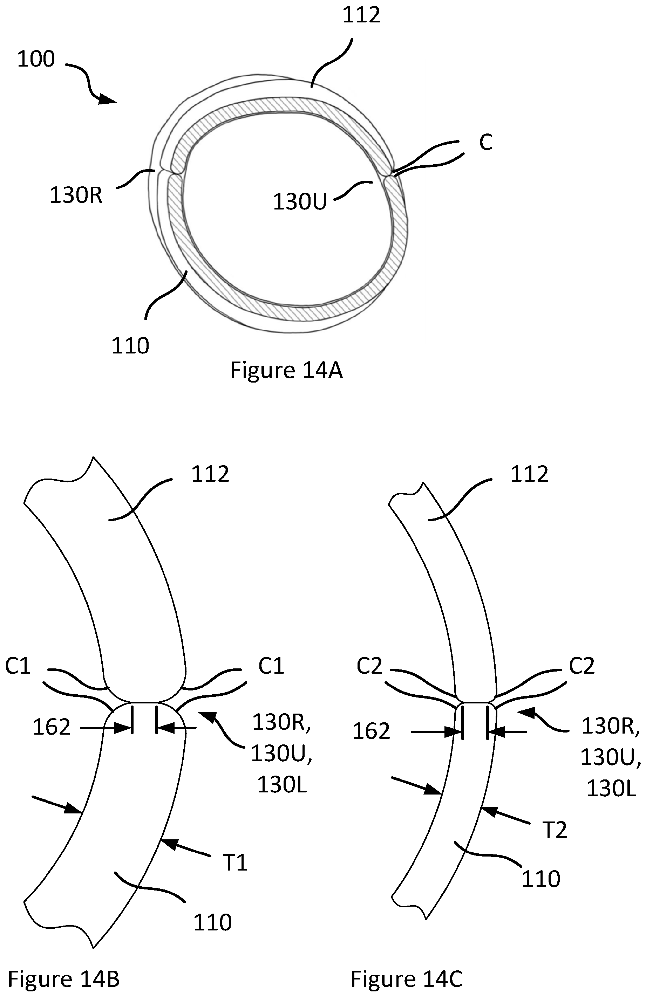

As shown in Figure 14A (and similarly in Figures 14B and 14C), at the radial join 130R and the ulnar join 130U (and at any lateral join 130L), the first cast portion 110 and the second cast portion 112 have curved edges C having a radius of curvature of at least 0.5 mm.

A curved edge C having a radius of curvature of at least 0.5 mm reduces the risk of the user's body hair becoming uncomfortably caught between the first cast portion 110 and the second cast portion 112 when closed into a closed configuration. A curved edge C having a radius of curvature of at least 0.5 mm reduces the risk of damage to the corresponding edges of the first cast portion 110 and the second cast portion 112 during assembly and disassembly (e.g. if brought together misaligned) and if exposed to impacts.

As shown in Figures 14B and 14C, at the radial join 130R and the ulnar join 130U (and at any lateral join 130L), the first cast portion 110 and the second cast portion 112 may have a first curved edge C1 where the cast 100 has a first layer thickness T1, and may have a second curved edge C2 where the cast 100 has a second layer thickness T2, where the second layer thickness T2 is at least 15% less than the first layer thickness T1, and wherein the radius of curvature of the second curved edge C2 is smaller than the radius of curvature of the first curved edge C1, and the radial join 130R and the ulnar join 130U have a mating face width 162 of at least 3 mm.

A minimum mating face width 162 of at least 3 mm provides resilient mating faces at the radial join 130R and the ulnar join 130U (and at any lateral join 130L), reducing the risk of damage to the corresponding edges of the first cast portion 110 and the second cast portion 112 during assembly and disassembly (e.g. if brought together misaligned) and if exposed to impacts.

The first cast portion 110 and the second cast portion 112 of the cast 100 may each be formed by a 3D printing process. For example the 3D printing may use liquid resin that is optically set by laser light, which is used to pattern successive layers, e.g. using stereolithography 3D printing to print thermoset polymer. In a further example, the 3D printing may set powdered polymer or may melt powdered metal (e.g. aluminium).

The cast 100 may be formed from material having a Youngs modulus of at least 600 MPa, e.g. at least 900 MPa. For example, the illustrated cast 100 may have a Youngs modulus of 1500 MPa.

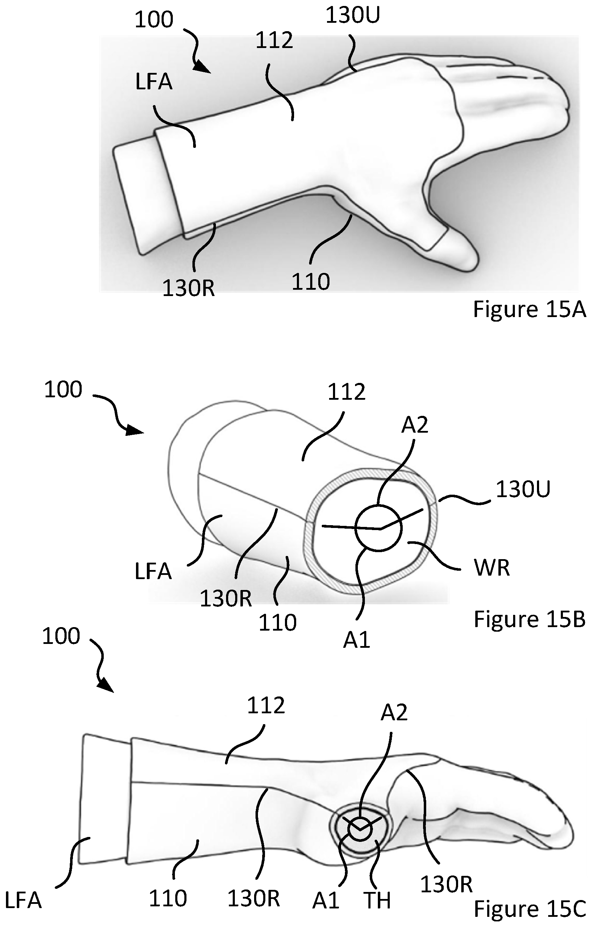

As is shown by Figures 1A to 1D and 15A, one of the first cast portion 110 and the second cast portion 112 may extend around a larger proportion of the lower forearm LFA and wrist WR of the user than the other of the first cast portion 110 and the second cast portion 112. Figure 15B shows a cross-section through the wrist WR of a user wearing the cast 100.

In the closed configuration, the first cast portion 110 (or the second cast portion 112) may extend through an angle A1 of 55% to 70% of 360 degrees (198 degrees to 252 degrees) around each of the lower forearm LFA and wrist WR of the user (e.g. may extend through an angle A1 of 60% of 360 degrees), with respect to the centre of the cast 100 at the lower forearm LFA and wrist WR. The complementary other cast portion 110, 112 may extend through an angle A2 of 30% to 45% of 360 degrees (108 degrees to 162 degrees) around each of the lower forearm LFA and wrist WR of the user, with respect to the centre of the cast 100 at the lower forearm LFA and wrist WR.

Similarly, where the first cast portion 110 and the second cast portion 112 extend along the thumb TH of the user, the first cast portion 110 (or the second cast portion 112) may extend through an angle A1 of 55% to 70% of 360 degrees (198 degrees to 252 degrees) around the thumb TH of the user, with respect to the centre of the cast 100 at the thumb TH. The complementary other cast portion 110, 112 may extend through an angle A2 of 30% to 45% of 360 degrees (108 degrees to 162 degrees) around the thumb TH of the user, with respect to the centre of the cast 100 at the thumb TH.

One of the first cast portion 110 and the second cast portion 112 extending around a larger proportion of the lower forearm LFA and wrist WR of the user than the other cast portion 110, 112 may facilitate fitting the cast 100, by enhancing the stability with which the user may holding that cast portion in place whilst connecting the other cast portion 112, 110. This may further enhance the ability for a user to fully assemble the cast using only their other (free) hand, enabling the cast 100 to be assembled without requiring the assistance of a carer or other third party.

One of the first cast portion 110 and the second cast portion 112 extending around a larger proportion of the lower forearm LFA and wrist WR of the user than the other cast portion 110, 112 may reduce the risk of skin and body hair becoming pinched between first cast portion 110 and the second cast portion 112, when assembled into the closed configuration.

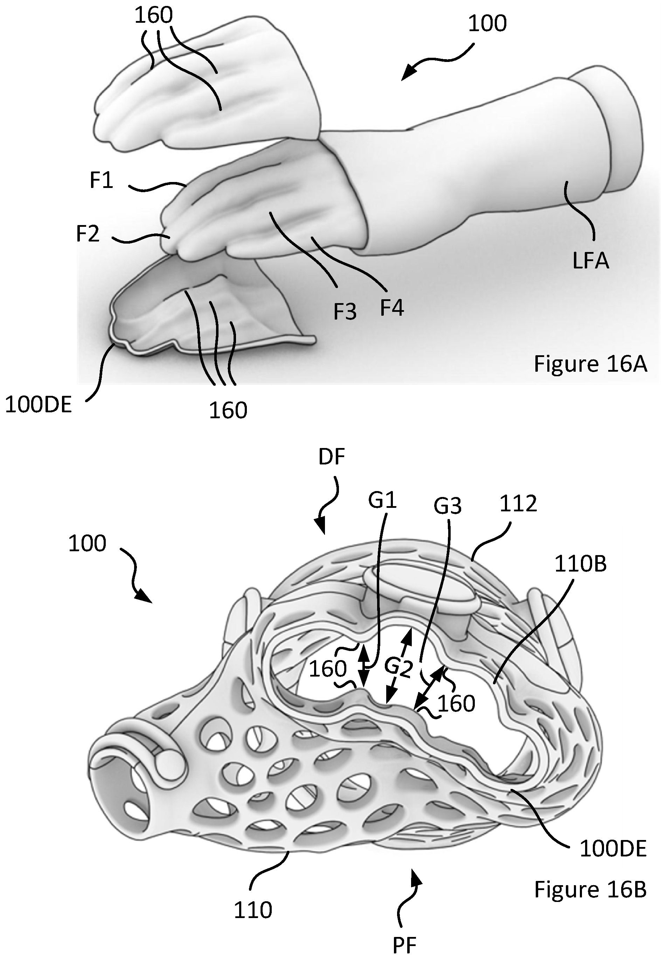

The first cast portion 110 and the second cast portion 112 may extend across both the planar face PF and the dorsal face DF of the finger metacarpophalangeal joints FMJ to a distal end 100DE that is at the finger proximal phalanx bones FPP or beyond. As shown in Figures 16A and 16B, adjacent at least the finger proximal phalanx bones FPP (and optionally adjacent the finger intermediate phalanx bones FIP and the finger distal phalanx bones FDP, as shown in Figure 16A), the interior surface of the cast 100 is provided with ridges 160 projecting inwardly between at least the index finger F1 and middle finger F2, and between the middle finger F2 and the ring finger F3. The minimal gap G1 between opposed ridges 160 aligned between the index finger F1 and the middle finger F2, and the minimal gap G3 between the opposed ridges 160 aligned between the middle finger F2 and the ring finger F3 are each at least 20% less than the maximal gap G2 between the opposed internal faces of the cast 100 aligned with the middle finger F2.

The inwardly projecting ridges 160 between the fingers FG may enhance user comfort in use, by distributing lateral forces across all of the fingers.

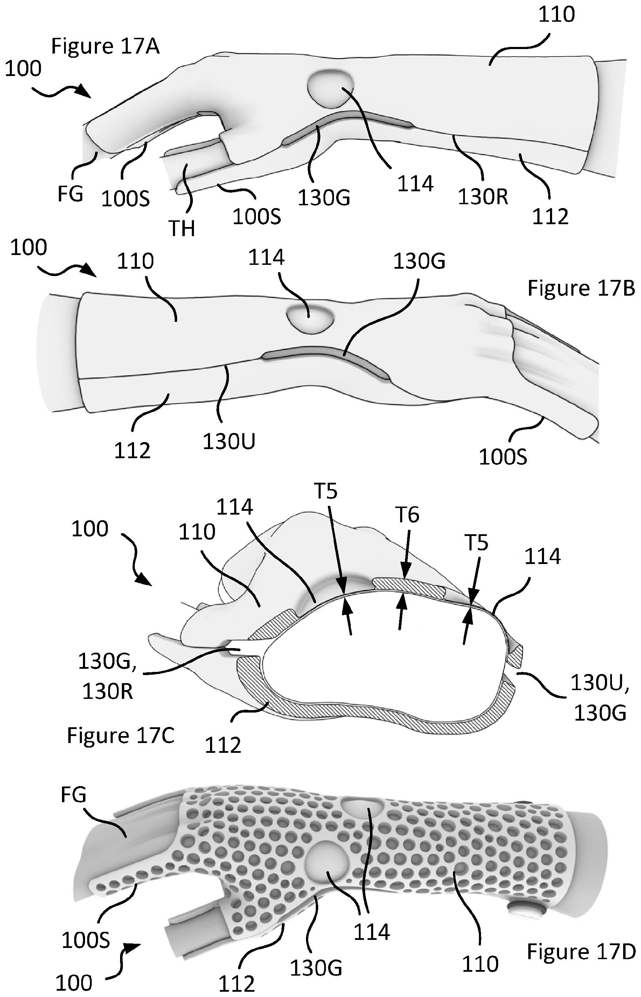

As shown in Figures 17A to 17D, along part of one or both of the radial join 130R and the ulnar join 130U, respectively along the radial side RS and the ulnar side US of the cast 100, one or both of the first cast portion 110 and the second cast portion 112 may remain spaced apart by an anti-pinch gap 130G, when the first cast portion 110 and the second cast portion 112 are in the closed configuration. The first cast portion 110 and the second cast portion 112 do not meet or abut at the anti-pinch gap 130G. Providing the anti-pinch gap 130G may reduce the risk of pinching loose skin between the first cast portion 110 and the second cast portion 112 during closure (e.g. during hinging together HT).

The anti-pinch gap 130G may be provided by recessing a portion of one or both of the first cast portion 110 and the second cast portion 112 from the opposed cast portion 112, 110.

An anti-pinch gap 130G may may be provided adjacent the wrist WR. Skin may be loose skin around the wrist WR, and providing an anti-pinch gap 130G adjacent the wrist WR may enhance patient comfort during closure of the cast 100.

The anti-pinch gap 130G may be at least 2mm wide. The anti-pinch gap 130G may be up to 10mm wide. The anti-pinch gap 130G may extend along at least 10% of the length of the respective radial join 130R or ulnar join 130U. The anti-pinch gap 130G may extend along up to 50% of the length of the respective radial join 130R or ulnar join 130U.

As shown in Figures 17A to 17D, the cast 100 may be provided with one or more comfort regions 114 that have enhanced flexibility relative to the part of the cast 100 surrounding the comfort regions 114. The enhanced flexibility may be provided by the cast 100 having a reduced thickness T5 compared with the thickness T6 of the surrounding part of the cast 100. Providing a comfort region 114 of reduced thickness T5 may enable the comfort region 114 to be produced with enhanced flexibility compared with the thickness T6 of the surrounding part of the cast 100, whilst being formed from the same material, enabling reduced complexity manufacturing of the cast 100 by 3D printing (or injection moulding).

The interior surface of the comfort region 114 may be flush with the interior surface of the surrounding part of the cast 100. Providing a flush interior finish around the comfort region 114 may reduce abrasion in use.

As shown in Figures 17A to 17D, comfort regions 114 may be provided adjacent one or both of the ulnar styloid process and the radial styloid process of the user. The ulnar styloid process and the radial styloid process may be regions of enhanced contact with the interior of the cast 100 in use, and providing corresponding comfort regions 114 of enhanced flexibility may enhance comfort in use.

As shown in Figures 17A, 17B and 17D, the cast 100 may be provided with a planar finger support 100S that extends at least partially along the length of the fingers FG and thumb TH, whilst the corresponding opposed dorsal regions of the fingers FG remain uncovered. The planar finger support 100S extends from the finger metacarpophalangeal joints FMJ, at least partially along the finger intermediate phalanx bones FIP, and may additionally extend at least partially along the finger proximal phalanx bones FPP. Providing the planar finger support 100S whilst leaving the opposed dorsal regions uncovered may stretch clenched muscles against their direction of involuntary clenching to provide a therapeutic benefit to the user, whilst enhancing ventilation to the fingers to enhance comfort.

The specific embodiments described below may be used in combination or interchangeably depending on the context. It is specifically intended in this disclosure that individual features of the cast that are disclosed in relation to any of the above figures may be considered more generally to represent features of the invention as a whole.

The figures provided herein are schematic.

Throughout the description and claims of this specification, the words "comprise" and "contain" and variations of them mean "including but not limited to", and they are not intended to (and do not) exclude other moieties, additives, components, integers, group of integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

## Claims

1. A cast (100) for supporting the lower forearm (LFA), wrist (WR) and at least a metacarpal portion (MP) of a hand of a user, comprising:
a first cast portion (110) that extends along a first face (PF, DF) of the lower forearm (LFA), wrist (WR) and metacarpal portion (MP) of the hand of the user, wherein the first face is the planar face (PF) or the dorsal face (DF); and
a second cast portion (120) that extends along the opposed second face (DF, PF) of the lower forearm (LFA), wrist (WR) and metacarpal portion (MP) of the hand of the user,
wherein the first cast portion (110) extends across both first and second faces (PF, DF) of one or more of the finger metacarpophalangeal joints (FMJ), the finger proximal phalanx bones (FPP), the finger intermediate phalanx bones (FIP) and the finger distal phalanx bones (FDP) of the hand of the user, and
wherein in a closed configuration, the first cast portion (110) and second cast portion (112) meet at a radial join (130R) and an ulnar join (130U) that respectively extend along the radial side (RS) and the ulnar side (US) of at least the lower forearm (LFA) and wrist (WR) of a user,
the first cast portion (110) and second cast portion (112) meet at a lateral join (130L) that extends between the radial join (130R) and the ulnar join (130U),
wherein one or more releasable hinge connectors (140H) are provided at the lateral join (130L) to releasably connect together the first cast portion (110) and the second cast portion (112), each of the one or more releasable hinge connectors (140H) comprising a first hinge part (140H1) and a cooperating second hinge part (140H2) being provided on different ones of the first cast portion (110) and the second cast portion (112).

2. A cast (100) for supporting the lower forearm (LFA), wrist (WR) and hand (MP, FG, TH) of a user,
comprising a releasably connectable first cast portion (110) and second cast portion (112) for enclosing the lower forearm (LFA), wrist (WR), and hand,
wherein in a closed configuration, the first cast portion (110) and second cast portion (112) abut along at least part of a radial join (130R) and an ulnar join (130U) that respectively extend along the radial side (RS) and the ulnar side (US) of at least the lower forearm (LFA) and wrist (WR) of a user,
wherein the distal end (100DE) of the cast (100) extends to, and provides a substantially rigid enclosure around, the fingertips (FT) and thumb-tip (TT) of a user.

3. The cast in accordance with claim 2, wherein the first cast portion (110) comprises:
a cast proximal portion (110PP) for enclosing the lower forearm (LFA), wrist (WR) and at least the metacarpal portion (MP) of the hand of the user; and
one or both of:
a finger distal portion (110FDP) that is releasably connectable to the cast proximal portion (110PP) for enclosing at least the finger proximal phalanx bones (FPP) and the finger intermediate phalanx bones (FIP) of the user; and
a thumb distal portion (110TDP) that is releasably connectable to the cast proximal portion (110PP) for enclosing at least the thumb distal phalanx bone (TDP) of the user.

4. The cast in accordance with claim 2 or claim 3, wherein in a closed configuration, the first cast portion (110) and second cast portion (112) abut along at least part of a radial join (130R) and an ulnar join (130U) that respectively extend along the radial side (RS) and the ulnar side (US) of at least the lower forearm (LFA) and wrist (WR) of a user, and optionally
wherein the first cast portion (110) and second cast portion (112) meet at a lateral join (130L) that extends between the radial join (130R) and the ulnar join (130U),
wherein one or more releasable hinge connectors (140H) are provided at the lateral join (130L) to releasably connect together the first cast portion (110) and the second cast portion (112), each of the one or more releasable hinge connectors (140H) comprising a first hinge part (140H1) and a cooperating second hinge part (140H2) being provided different ones of the first cast portion (110) and the second cast portion (112).

5. A cast (100) for supporting the lower forearm (LFA), wrist (WR) and at least the metacarpal portion (MP) of a hand of a user,
comprising a releasably connectable first cast portion (110) and second cast portion (112) for cooperatively enclosing the lower forearm (LFA), wrist (WR) and metacarpal portion (MP) of the hand,
wherein in a closed configuration, the first cast portion (110) and second cast portion (112) abut along at least part of a radial join (130R) and an ulnar join (130U) that respectively extend along the radial side (RS) and the ulnar side (US) of at least the lower forearm (LFA) and wrist (WR) of a user.

6. The cast in accordance with claim 6, wherein the first cast portion (110) extends across both the planar face (PF) and the dorsal face (DF) of the finger metacarpophalangeal joints (FMJ), the finger proximal phalanx bones (FPP), the finger intermediate phalanx bones (FIP) and the finger distal phalanx bones (FDP) of the hand of the user, and
the first cast portion (110) and second cast portion (112) meet at a lateral join (130L) that extends between the radial join (130R) and the ulnar join (130U), and optionally
wherein one or more releasable hinge connectors (140H) are provided at the lateral join (130L) to releasably connect together the first cast portion (110) and the second cast portion (112), each of the one or more releasable hinge connectors (140H) comprising a first hinge part (140H1) and a cooperating second hinge part (140H2) being provided different ones of the first cast portion (110) and the second cast portion (112).

7. The cast in accordance with one of claims 1, 4 and 6, wherein one or more releasable hinge connectors (140H) are provided at the lateral join (130L) to releasably connect together the first cast portion (110) and the second cast portion (112), wherein, in each of the one or more releasable hinge connectors (140H):
the first hinge part (140H1) comprises a projecting hinge tongue (140HT) for engaging into the second hinge part (140H2);
the second hinge part (140H2) comprises a tapered hinge groove (140HG) with an opening for receiving the projecting hinge tongue (140HT) of the first hinge part (140H1), in which the tapered hinge groove (140HG) increases in width (140HW) away from the opening.

8. The cast in accordance with one of claims 1, 4, 6 and 7, wherein one or more releasable hinge connectors (140H) are provided at the lateral join (130L) to releasably connect together the first cast portion (110) and the second cast portion (112), wherein, when the first cast portion (110) and the second cast portion (112) are in a connected configuration, the first hinge part (140H1) abuts the second hinge part (140H2) in an inner part of each releasable hinge connector (140H) that is proximate the interior of the cast (100), and the first hinge part (140H1) is spaced apart from the second hinge part (140H2) by a hinging gap (146) in an outer part of each releasable hinge connector (140H) that is remote from the interior of the cast (100).

9. The cast in accordance with any preceding claim, comprising an anti-pinch gap (130G) along part of one or both of the radial join (130R) and the ulnar join (130U), wherein the first cast portion (110) and the second cast portion (112) are configured to remain spaced apart along the anti-pinch gap (130G), when the first cast portion (110) and the second cast portion (112) are in the closed configuration.

10. The cast in accordance with any preceding claim, comprising a comfort region (114) that have enhanced flexibility relative to the part of the cast (100) surrounding the comfort region (114).

11. The cast in accordance with any preceding claim, wherein one or more releasable alignment connectors (140A) are provided at each of the radial join (130R) and the ulnar join (130U) to releasably connect together the first cast portion (110) and the second cast portion (112), and
wherein each of the releasable alignment connectors (140A) comprises a first alignment connector part (140A1) having a projecting alignment tongue (140AT), and a complementary second alignment connector part (140A2) having an alignment groove (140AG), the first alignment connector part (140A1) and the second alignment connector part (140A2) being provided on different ones of the first cast portion (110) and the second cast portion (112), and optionally one or both of:
(i) wherein, in each of the one or more releasable alignment connectors (140A), the first alignment connector part (140A1) comprises a projecting alignment tongue (140AT) for engaging into the second side connector part (140A2), and the second alignment connector part (140A2) comprises a tapered alignment groove (140AG) with an opening for receiving the projecting alignment tongue (140AT) of the first alignment connector part (140A1), in which the tapered alignment groove (140AG) narrows in width away from the opening; and
(ii) wherein one or each releasable alignment connector (140A) comprises a circumferential connector groove (142) for receiving a connection band (144) for releasably biasing together parts (140A1, 140A2) of one or each releasable alignment connector (140A).

12. The cast in accordance with claim 7, wherein a drainage hole (149) is provided, extending from the tapered hinge groove (140HG) or tapered alignment groove (140AG), to an exterior surface of the second hinge part (140H2) or second alignment connector part (140A2), extending parallel to an insertion direction (ID) of a complementary projecting hinge tongue (140HT) or projecting alignment tongue (140AT).

13. The cast in accordance with any preceding claim, comprising:
(i) wherein the cast (100) has a first layer thickness (T1) for covering the lower forearm (LFA) and wrist (WR), and a thinner second layer thickness (T2) for covering at least the finger metacarpophalangeal joints (FMJ), wherein second layer thickness (T2) is at least 15% less at the finger metacarpophalangeal joints (FMJ); or
(ii) wherein centrally along the planar face (PF) the cast (100) has a third layer thickness (T3) and centrally along the dorsal face (DF) the cast (100) has a fourth layer thickness (T4), wherein the fourth layer thickness (T4) is at least 15% less than the third layer thickness (T3).

14. The cast in accordance with any preceding claim, wherein, at the radial join (130R) and the ulnar join (130U), the first cast portion (110) and the second cast portion (112) have a first curved edge (C1) where the cast (100) has a first layer thickness (T1) and a second curved edge (C2) where the cast (100) has a second layer thickness (T2), wherein the second layer thickness (T2) is at least 15% less than the first layer thickness (T1), wherein the radius of curvature of the second curved edge (C2) is smaller than the radius of curvature of the first curved edge (C1), and the radial join (130R) and the ulnar join (130U) have a mating face width (162) of at least 3 mm.

15. The cast in accordance with any preceding claim, wherein the first cast portion (110) and the second cast portion (112) extend across both the planar face (PF) and the dorsal face (DF) of the finger metacarpophalangeal joints (FMJ) to a distal end (100DE) that is at the finger proximal phalanx bones (FPP) or beyond, and
adjacent at least the finger proximal phalanx bones (FPP), the interior surface of the cast (100) is provided with ridges (160) projecting inwardly between at least the index finger (F1), middle finger (F2) and ring finger (F3) of a user, wherein the minimal gap (G1) between opposed ridges (160) aligned between the index finger (F1) and the middle finger (F2), and the minimal gap (G3) between the opposed ridges (160) aligned between the middle finger (F2) and the ring finger (F3) are each at least 20% less than the maximal gap (G2) between the opposed internal faces of the cast (100) aligned with the middle finger (F2).
